(19) Europäisches Patentamt
European Patent Office
Office européen des brevets

(11) EP 3 677 581 A1

(12) **EUROPEAN PATENT APPLICATION**
published in accordance with Art. 153(4) EPC

(43) Date of publication:
**08.07.2020 Bulletin 2020/28**

(21) Application number: **18850914.5**

(22) Date of filing: **03.09.2018**

(51) Int Cl.:
***C07D 413/12*** *(2006.01)*

(86) International application number:
**PCT/CN2018/103846**

(87) International publication number:
**WO 2019/042451 (07.03.2019 Gazette 2019/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.09.2017 CN 201710780917**
**29.12.2017 CN 201711478670**

(71) Applicant: **Nanjing Transthera Biosciences Co. Ltd.**
**Jiangsu 210032 (CN)**

(72) Inventors:
- **WU, Frank**
**Jiangsu 210032 (CN)**
- **LI, Lin**
**Jiangsu 210032 (CN)**

(74) Representative: **De Vries & Metman**
**Overschiestraat 180**
**1062 XK Amsterdam (NL)**

# (54) DEUTERATED INDOLEAMINE 2,3-DIOXYGENASE INHIBITOR AND APPLICATION THEREOF

(57) The present invention belongs to the technical field of medicine, and particularly relates to a compound represented by Formula I, a pharmaceutically acceptable salt thereof, and a stereoisomer thereof, $R^4$, $R^{4'}$, $R^5$, $R^{5'}$, $R^6$, $R^7$, $R^{1a}$, $R^{2a}$, m, n, $t_1$, and $t_2$ are defined in the description; the present invention further relates to a Crystalline Form of the compound represented by Formula I, and the present invention also relates to a pharmaceutical preparation, a pharmaceutical composition, a preparation method of these compounds and Crystalline Forms thereof, as well as a use thereof in manufacture of a medicament for treating a related disease mediated by abnormality of indoleamine 2,3-dioxygenase (IDO).

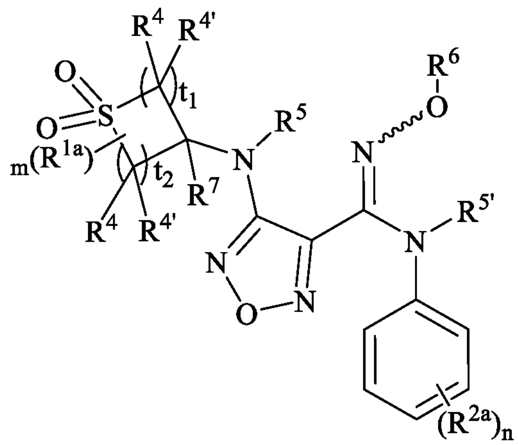

I



Printed by Jouve, 75001 PARIS (FR)

## Description

### Technical Field

The present invention belongs to the technical field of medicine, and relates to a compound as a deuterated indoleamine 2, 3-dioxygenase (IDO) inhibitor, a pharmaceutically acceptable salt and stereoisomer thereof, a pharmaceutical preparation and pharmaceutical composition of these compounds, and a use thereof in manufacture of a medicament for the treatment of a related disease mediated by abnormality of indoleamine 2,3-dioxygenase (IDO).

### Background Art

Tryptophan (Trp) is an essential amino acid in the body, and is useful in biosynthesis of proteins, niacin and neurotransmitter 5-hydroxytryptamine (serotonin) in the body. Indoleamine 2,3-dioxygenase (IDO) is a monomeric enzyme containing heme in cells, and is widely distributed in many tissues and cells of humans and animals. IDO is capable of catalyzing the first step reaction of indole epoxidative cleavage of L-tryptophan to produce kynurenine, and this step is also a key rate-limiting reaction. IDO is expressed at a low level in the normal state of the body, while an abnormally high expression may be detected in a disease state.

It is found in some research and development that IDO is involved in many pathological processes, including chronic infection, HIV-infection, AIDS, autoimmune disease, Alzheimer's disease, and the like. In addition, IDO can also be an important target for small molecule regulation in the field of anti-tumor immunotherapy. The regulation of immune system mainly includes the following aspects: (1) high IDO expression may lead to local tryptophan depletion of cells, because T cells are particularly sensitive to tryptophan depletion, so when the tryptophan concentration decreases, the proliferation of T cells will stagnate in G1 phase; (2) IDO-dependent tryptophan degradation leads to an increase in kynurenine and induces oxygen free radical-mediated T cell apoptosis; (3) up-regulation of IDO expression in dendritic cells enhances local regulatory T-cell (Treg)-mediated immunosuppression by degrading local tryptophan, thereby promoting the peripheral immune tolerance to tumor-specific antigens in the body.

Based on the important effects of IDO on the formation and maintenance of tumor immune tolerance, IDO inhibitors are expected to become a new type of adjuvant tumor immunotherapy drugs. However, there is no IDO inhibitor drug currently on the market, so that a new effective IDO inhibitor is currently in urgent need.

The principle of technology for deuterating drug is to improve a drug involved in the carbon-hydrogen bond cleavage in drug metabolism in vivo. Deuterium is also known as deuterohydrogen, and the bond between deuterium and carbon is stronger than the bond between ordinary hydrogen and carbon. One of the key metabolic clearance pathways in human body relies on the cleavage of carbon-hydrogen bond. In many cases, the replacement of a few of hydrogen atoms with deuterium in a drug could slow down the decomposition of carbon-deuterium bonds by enzymes, so that the drug may stay longer in the body while reducing the formation of toxic metabolites. Generally speaking, compared with non-deuterated drugs, deuterated drugs usually have the effects of reducing toxic side effects, increasing drug stability, enhancing efficacy, and extending the biological half-life of drugs.

The present invention is dedicated to studying a series of new and efficient deuterated IDO inhibitors, which have good properties as a drug and can be used to prevent and/or treat Alzheimer's disease, cataract, infection associated to cellular immune activation, autoimmune disease, AIDS, Cancer, depression and other diseases, which are caused by abnormal tryptophan metabolism.

### Contents of the Invention

The technical problem to be solved by the present invention is to provide a new type of IDO inhibitors, and such compounds have good inhibitory activity on IDO and better pharmacokinetic stability than non-deuterated compounds, can increase drug stability, enhance efficacy, extend drug half-life, and can be used to treat a disease mediated by IDO abnormality.

The present invention provides the following technical solutions:
A compound of Formula I, a pharmaceutically acceptable salt thereof and a stereoisomer thereof:

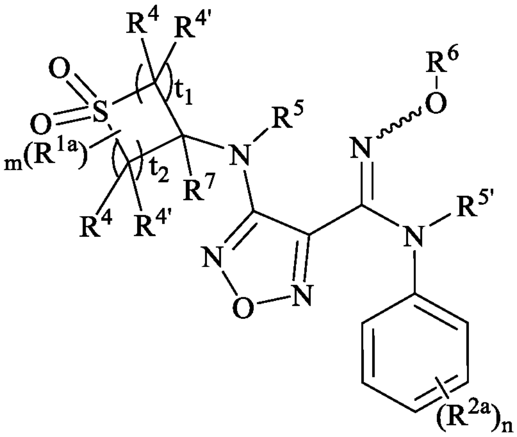

wherein,

$\sim$ represents a cis isomer, a trans isomer, or a mixture of cis and trans isomers;

$R^4$ and $R^{4'}$ are independently selected from hydrogen, deuterium, $C_{1-6}$ alkyl, or $C_{1-6}$ alkyl deuterated by one or more deuterium atoms;

$R^5$, $R^{5'}$, $R^6$, $R^7$ are independently selected from hydrogen or deuterium;

each $R^{1a}$ is independently selected from hydrogen, deuterium, halogen, $C_{1-6}$ alkyl, or $C_{1-6}$ alkyl deuterated by one or more deuterium atoms;

each $R^{2a}$ is independently selected from hydrogen, deuterium, halogen, $C_{1-6}$ alkyl, or $C_{1-6}$ alkyl deuterated by one or more deuterium atoms;

m is 0, 1 or 2;

n is 0, 1, 2, 3, 4 or 5;

$t_1$ and $t_2$ are independently 0, 1, 2, 3, and $t_1$ and $t_2$ are not 0 at the same time.

In another preferred embodiment, the compound of Formula I, a pharmaceutically acceptable salt thereof, and a stereoisomer thereof:

$t_1$ is 0, and $t_2$ is 1, 2 or 3; or

$t_1$ is 1, and $t_2$ is 0, 1, 2 or 3; or

$t_1$ is 2, and $t_2$ is 0, 1, 2, or 3; or

$t_1$ is 3, and $t_2$ is 0, 1,2 or 3; or

$t_1$ is 1, 2 or 3, and $t_2$ is 0; or

$t_1$ is 0, 1, 2 or 3, and $t_2$ is 1; or

$t_1$ is 0, 1, 2 or 3, and $t_2$ is 2; or

$t_1$ is 0, 1, 2 or 3, and $t_2$ is 3; or

In another preferred embodiment, the compound of Formula I, a pharmaceutically acceptable salt thereof, and a stereoisomer thereof:

$t_1$ is 1, and $t_2$ is 1 or 2; or

$t_1$ is 2, and $t_2$ is 1 or 2.

In another preferred embodiment, the compound of Formula I, a pharmaceutically acceptable salt thereof, and a stereoisomer thereof further have a structure represented by Formula II:

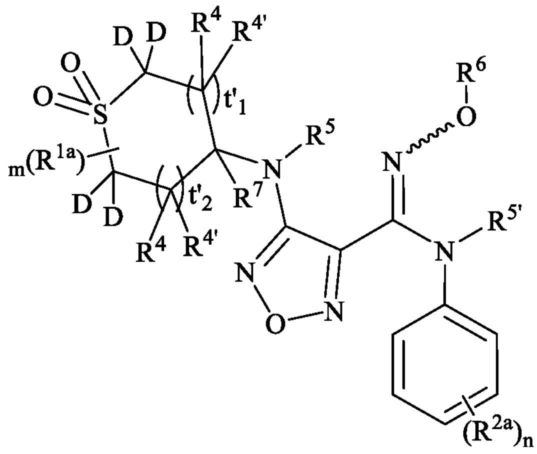

II

wherein, ∿ represents a cis isomer, a trans isomer, or a mixture of cis and trans isomers;
$R^4$ and $R^{4'}$ are independently selected from hydrogen or deuterium;
$R^5$, $R^{5'}$, $R^6$, $R^7$ are independently selected from hydrogen or deuterium;
each $R^{1a}$ is independently selected from hydrogen or $C_{1-6}$ alkyl;
each $R^{2a}$ is independently selected from hydrogen, deuterium, halogen or $C_{1-6}$ alkyl;
m is 0, 1 or 2;
n is 0, 1, 2, 3, 4 or 5;
$t'_1$ and $t'_2$ are each independently 0, 1 or 2.

In another preferred embodiment, the compound of Formula II, a pharmaceutically acceptable salt thereof, and a stereoisomer thereof:

wherein, ∿ represents a cis isomer, a trans isomer, or a mixture of cis and trans isomers;
$R^4$ and $R^{4'}$ are independently selected from hydrogen or deuterium;
$R^5$, $R^{5'}$, $R^6$, $R^7$ are independently selected from hydrogen;
each $R^{1a}$ is independently selected from hydrogen;
each $R^{2a}$ is independently selected from hydrogen, deuterium or halogen;
m is 0;
n is 0, 1 or 2, and more preferably 2.
$t'_1$ and $t'_2$ are each independently 0, 1 or 2.

In another preferred embodiment, the compound of Formula II, a pharmaceutically acceptable salt thereof, and a stereoisomer thereof:

$t'_1$ is 0, and $t'_2$ is 0, 1 or 2; or

$t'_1$ is 1, and $t'_2$ is 0, 1 or 2; or

$t'_1$ is 2, and $t'_2$ is 0, 1 or 2; or

$t'_1$ is 0, 1 or 2, and $t'_2$ is 0; or

$t'_1$ is 0, 1 or 2, and $t'_2$ is 1; or

t'$_1$ is 0, 1 or 2, and t'$_2$ is 2; or

In another preferred embodiment, the compound of Formula II, a pharmaceutically acceptable salt thereof, and a stereoisomer thereof:

t'$_1$ is 0, and t'$_2$ is 0 or 1; or

t'$_1$ is 1, and t'$_2$ is 0 or 1; or

t'$_1$ is 2, and t'$_2$ is 0 or 1.

In another preferred embodiment, the compound of Formula I comprises at least one deuterium atom, more preferably comprises two deuterium atoms, and even more preferably comprises four deuterium atoms.

In another preferred embodiment, the compound of Formula II comprises at least four deuterium atoms.

In the above embodiments or technical solutions, each position indicated as deuterium has at least 50% deuterium enrichment, preferably at least 65% deuterium enrichment, preferably at least 75% deuterium enrichment, preferably at least 85% deuterium enrichment, more preferably 90%, at least 90% deuterium enrichment, more preferably 95%, at least 95% deuterium enrichment, more preferably at least 98% deuterium enrichment, more preferably at least 99% deuterium enrichment.

According to one aspect of the present invention, the following compounds, pharmaceutically acceptable salts thereof, or stereoisomers thereof are provided:

| No. | Structural Formula | No. | Structural Formula |
|---|---|---|---|
| 1 | 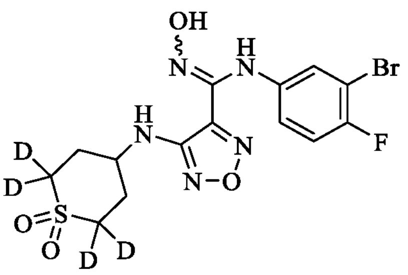 | 2 | 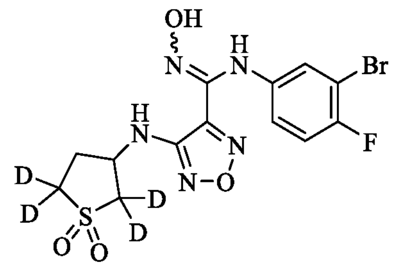 |
| 3 | 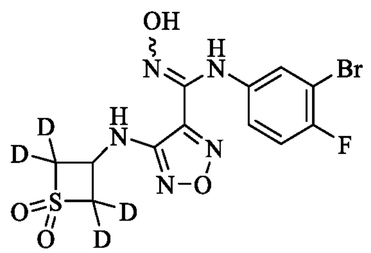 | 4 | 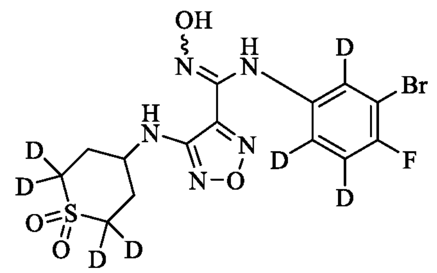 |
| 5 | 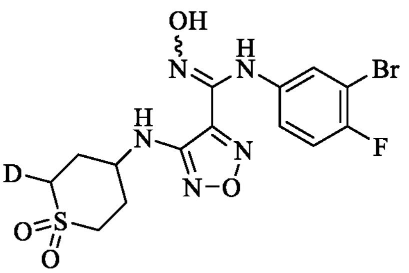 | 6 | 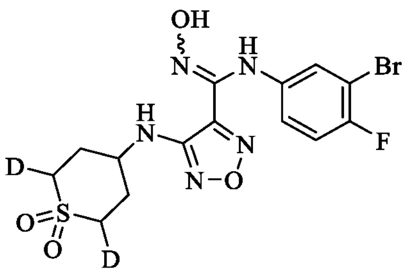 |

(continued)

| No. | Structural Formula | No. | Structural Formula |
|---|---|---|---|
| 7 | 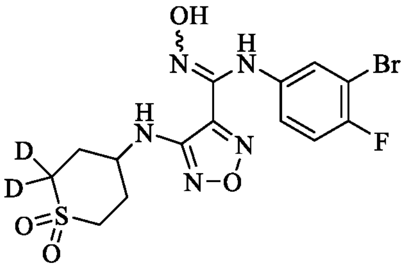 | 8 | 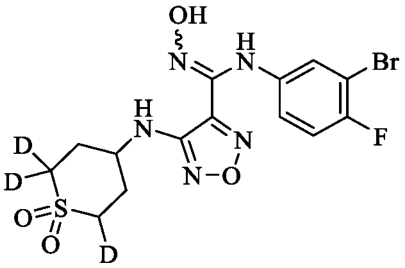 |

Another aspect of the present invention provides a crystalline compound. For example, another aspect of the present invention provides Crystalline Form A and Crystalline Form B of Compound 1.

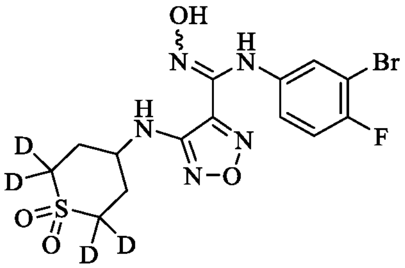

Compound 1.

**Crystal Form A of Compound 1**

In one embodiment, Crystalline Form A of Compound 1 shows an X-ray powder diffraction pattern including peaks at the following diffraction angles (2θ): 8.8±0.2°, 18.0±0.2°, 19.5±0.2°, 22.4±0.2°, 23.1±0.2°, 23.8±0.2°, 27.8±0.2°.

In another embodiment, Crystalline Form A of Compound 1 shows an X-ray powder diffraction pattern including peaks at the following diffraction angles (2θ): further including 8.8±0.2°, 11.9±0.2°, 17.6±0.2°, 18.0±0.2°, 18.7±0.2°, 19.5±0.2°, 22.4±0.2°, 23.1±0.2°, 23.8±0.2°, 25.1±0.2°, 26.8±0.2°, 27.8±0.2°, 30.0±0.2°.

In yet a further embodiment, Crystalline Form A can be further characterized as having substantially the X-ray powder diffraction pattern as shown in Figure 1.

The X-ray powder diffraction patterns can be obtained using CuKa radiation. The temperature at which the X-ray powder diffraction patterns are obtained may be at, for example, 25±2 degrees Celsius.

Crystal Form A can be further characterized by having a melting point starting at about 218-221°C (preferably 219-220°C). Still further, the crystalline compound can be further characterized by a differential scanning calorimetry curve substantially the same as that shown in Figure 2.

**Crystal Form B of Compound 1**

In one embodiment, Crystalline Form B of Compound 1 shows an X-ray powder diffraction pattern including peaks at the following diffraction angles (2θ): 16.2±0.2°, 18.0±0.2°, 21.1±0.2°, 21.6±0.2°, 21.8±0.2°, 23.7±0.2°, 23.9±0.2°, 26.2±0.2°.

In one embodiment, Crystalline Form B of Compound 1 shows an X-ray powder diffraction pattern including peaks at the following diffraction angles (2θ): further including 10.7±0.2°, 15.8±0.2°, 16.2±0.2°, 16.7±0.2°, 18.0±0.2°, 19.3±0.2°, 20.1±0.2°, 21.1±0.2°, 21.6±0.2°, 21.8±0.2°, 22.5±0.2°, 23.2±0.2°, 23.7±0.2°, 23.9±0.2°, 26.2±0.2°.

In yet a further embodiment, Crystalline Form B can be further characterized as having substantially the X-ray powder diffraction pattern as shown in Figure 3.

The X-ray powder diffraction patterns can be obtained using CuKa radiation. The temperature at which the X-ray powder diffraction patterns are obtained may be at, for example, 25±2 degrees Celsius.

Crystal Form B can be further characterized by having a melting point starting at about 236-239°C (preferably 236-238°C). Still further, the crystalline compound can be further characterized by a differential scanning calorimetry curve substantially the same as that shown in Figure 4.

Another technical solution of the present invention is to provide a pharmaceutical composition comprising the compound represented by Formula I and Formula II, a pharmaceutically acceptable salt thereof, and a stereoisomer thereof, which may optionally comprises one or more pharmaceutically acceptable carriers, and may be prepared into any pharmaceutically acceptable dosage form. The pharmaceutical composition may be administered to a patient or subject in need of treatment by any suitable administration mode, such as oral, parenteral, rectal, or pulmonary administration. When used for oral administration, the pharmaceutical composition may be formulated into a conventional solid preparation, such as tablet, capsule, pill, granule, etc.; or may be formulated into an oral liquid preparation, such as oral solution, oral suspension, syrups, etc. When making an oral preparation, a suitable filler, binder, disintegrating agent, lubricant and the like may be added. When used for parenteral administration, the pharmaceutical composition may be formulated into an injection, including injection solution, sterile powder for injection, and concentrated solution for injection. When making an injection, it may be produced by a conventional method in the existing pharmaceutical field. When an injection is prepared, an additional agent may not be added, or a suitable additional agent may be added according to the properties of the drug. When used for rectal administration, the pharmaceutical composition may be formulated into a suppository and the like. When used for pulmonary administration, the pharmaceutical composition may be formulated into an inhalant, a spray and the like.

Another technical solution of the present invention is to provide a use of the compound represented by Formula I and Formula II, a pharmaceutically acceptable salt thereof, and a stereoisomer thereof in manufacture of a medicament for treating a disease mediated by IDO abnormality, especially for treating a tumor-specific immunosuppression associated with a cancer.

The disease mediated by IDO abnormality is an infectious disease, a nervous system disease, a cancer or a non-cancerous proliferative disease. The infectious disease includes diseases induced by viruses such as influenza virus, hepatitis C virus (HCV), human papilloma virus (HPV), cytomegalovirus (CMV), E-B virus (EBV), polio virus, varicella zoster virus, Coxsackie virus, human immunodeficiency virus (HIV); the neurological disease includes: Alzheimer's disease, depression; the cancer includes lung cancer, squamous cell carcinoma, bladder cancer, gastric cancer, ovarian cancer, peritoneal cancer, breast cancer, breast ductal cancer, head and neck cancer, endometrial cancer, uterine cancer, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharynx cancer, multiple myeloma, leukemia, non-Hodgkin's lymphoma, colonic villous adenoma, melanoma, cell tumor and sarcoma, and myelodysplastic syndrome.

## Detailed description of the invention

The "halogen" in the present invention refers to fluorine, chlorine, bromine, iodine and the like, and preferably a fluorine atom or a bromine atom.

The term "$C_{1-6}$ alkyl" in the present invention refers to a straight or branched alkyl derived from a hydrocarbon moiety containing 1-6 carbon atoms by removing one hydrogen atom, such as methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl , 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, and 1-methyl-2-methylpropyl and the like. The term "$C_{1-4}$ alkyl" refers to the above examples containing 1-4 carbon atoms.

As used herein, "deuterated" refers to the replacement of one or more hydrogen atoms in a compound or group with deuterium atom(s).

The term "deuterium enrichment" according to the present invention is a term well-known in the art and refers to the percentage of hydrogen atoms replaced by deuterium atoms. In the present invention, the term "deuterium enrichment" is also referred to as "deuteration rate".

The "pharmaceutically acceptable salt" in the present invention refers to a pharmaceutically acceptable addition salt of acids and bases of the compound of Formula I or Formula II. When the compound contains an acidic functional group (such as -COOH, -OH, $-SO_3H$, etc.), its salt can be formed with an appropriate inorganic or organic cation (base), comprising a salt formed with an alkaline metal or alkaline earth metal, an ammonium salt, and a salt formed with a nitrogen-containing organic base; when the compound contains a basic functional group (such as -NH2, etc.), its salt can be formed with an appropriate inorganic or organic anion (acid), comprising an inorganic acid salt and an organic acid salt. Such "pharmaceutically acceptable salt" includes, but is not limited to, a salt of an acid: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid, $HOOC-(CH_2)_n-COOH$ (wherein n is 0 to 4)), etc.; a salt of an alkali: sodium, potassium, calcium, and ammonium, etc.

The "stereoisomer" in the present invention refers to an enantiomer produced when the compound of Formula I or Formula II has an asymmetric carbon atom; when the compound has a carbon-carbon double bond or a cyclic

structure, cis-trans isomers may be produced; when the compound has ketone or oxime group, tautomers may be produced; and all enantiomers, diastereomers, racemates, cis-trans isomers, tautomers, geometric isomers, epimers of the compound of Formula I or Formula II and the mixtures thereof fall into the scope of the present invention.

For example:

When there is

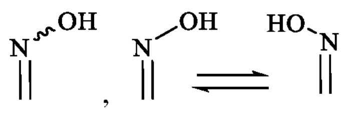


may be produced.

When there is

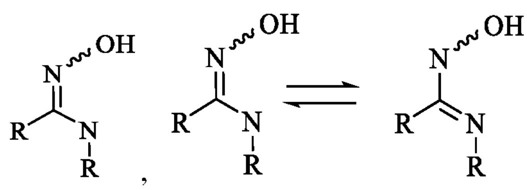


may be produced.

The compound of the present invention is prepared by preparation steps as follows:

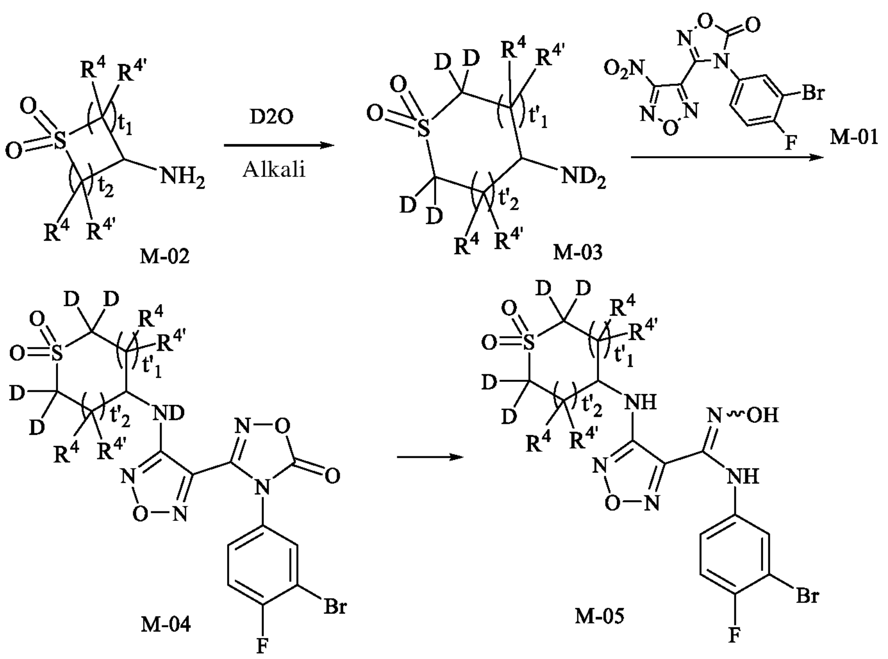


wherein, $R^4$, $R^{4'}$, $R^5$, $R^{5'}$, $t_1$, $t_2$, $t_1'$, $t_2'$ are as defined above.

Step 1: M-02 is dissolved in deuteroxide, an alkali is added, the reaction is carried out at reflux for more than 2 hours (preferably more than 3 hours), the deuteration rate is detected by using NMR, and deuteroxide is added repeatedly until the deuteration rate detected by NMR is above 75% (preferably 90% or more, more preferably 99% or more) to obtain a mother liquor of M-03.

Step 2: Under the protection of nitrogen, a 2-methyltetrahydrofuran solution (which can be replaced by tetrahydrofuran, dimethylsulfoxide, ethyl acetate, acetonitrile, dimethylacetamide solution) of M-01 is added dropwise to a deuteroxide solution of M-03, when the reaction is carried out for more than 1 hour (preferably 2 hours) and the pH is measured to 7-12 (preferably 8-9), anhydrous sodium carbonate (which can be replaced by anhydrous sodium bicarbonate, anhydrous sodium hydroxide, imidazole, N,N-diisopropylethylamine (DIEA)) is added, the reaction is carried out

at 20-60°C (preferably 25-35°C) for more than 10 hours (preferably 15-20 hours), the reaction solution is subjected to suction filtration, then washed with water, acid aqueous solution (which is adjusted with hydrochloric acid, sulfuric acid, etc., to have an acidic pH, preferably pH 3-4) and anhydrous methanol in sequence, and dried to obtain M-04.

Step 3: Under the protection of nitrogen, a solution of M-04 in tetrahydrofuran (which can be replaced by dimethyltetrahydrofuran, dimethylformamide, N,N-diisopropylethylamine) is added, an aqueous solution of methylamine is added, and the reaction is carried out at reflux for 0.1-1 hour, the reaction solution is subjected to suction filtration, rinsed with ethyl acetate, the filtrate is washed with a mixed solution of concentrated hydrochloric acid and brine, , the organic phase is subjected to suction filtration and concentrated under reduced pressure to obtain M-05.

The "alkali" refers to a basic substance such as sodium hydroxide, potassium hydroxide, calcium hydroxide, and the like.

Under alkaline conditions, exchange of D and H occurs to reach equilibrium. In order to increase the deuteration rate, deuteration is required to be performed for several times. When the deuteration rate is more than 99%, the purity of the obtained M-05 is not less than 96%.

| | Deuterated once | Deuterated twice | Deuterated 3 times | Deuterated 4 times | Deuterated 5 times |
|---|---|---|---|---|---|
| Deuteration rate | At least 65% | At least 85% | At least 90% | At least 95% | At least 99% |

**Preparation of Crystalline Form A of Compound 1 of the present invention**

The crude product of Compound 1 obtained in the preparation process is slurried in an organic solvent at low temperature, filtered, and dried to obtain Crystalline Form A.

The low temperature refers to 10°C to 20°C.

The organic solvent refers to one or more of ethyl acetate, anhydrous methanol, absolute ethanol, isopropanol, dichloromethane, dichloroethane, methyl tert-butyl ether, tetrahydrofuran, and toluene, preferably ethyl acetate, and anhydrous methanol.

**Preparation of Crystalline Form B of Compound 1 of the present invention**

The crude product of Compound 1 obtained in the preparation process is slurried under reflux in an organic solvent, and then slurried at 15-30°C, filtered, and dried to obtain Crystalline Form B.

The organic solvent refers to one or more of ethyl acetate, anhydrous methanol, absolute ethanol, isopropanol, acetone, acetonitrile, dichloromethane, dichloroethane, methyl tert-butyl ether, tetrahydrofuran and toluene, preferably ethyl acetate, anhydrous methanol and acetone.

**Brief Description of the Drawings**

In order to more clearly explain the examples of the present invention and the technical solutions of the prior art, the drawings used in the examples and the prior art are briefly described below. Obviously, the drawings in the following description are only for some examples of the present invention, and for those skilled in the art, other drawings can be obtained based on these drawings without creative efforts.

Figure 1 shows an X-ray powder diffraction (XRPD) pattern of Crystalline Form A of Compound 1.

Figure 2 shows a differential scanning thermal analysis (DSC) spectrum of Crystalline Form A of Compound 1.

Figure 3 shows an X-ray powder diffraction (XRPD) pattern of Crystalline Form B of Compound 1.

Figure 4 shows a differential scanning thermal analysis (DSC) spectrum of Crystalline Form B of Compound 1.

Figure 5 shows a chart of deuteration rate detection for Compound 1.

Figure 6 shows an X-ray powder diffraction (XRPD) pattern of Crystalline Form A of Compound 1 after leaving at a high temperature of 105°C for 3 days.

Figure 7 shows an X-ray powder diffraction (XRPD) spectrum of Crystalline Form A of Compound 1 after leaving at a high humidity of RH 92.5% for 3 days.

Figure 8 shows an X-ray powder diffraction (XRPD) spectrum of Crystalline Form B of Compound 1 after leaving at a high temperature of 60°C for 10 days.

Figure 9 shows an X-ray powder diffraction (XRPD) spectrum of Crystalline Form B of Compound 1 after leaving at a high humidity of RH 92.5% for 10 days.

## Specific Models for Carrying Out the Invention

**Example 1: Synthesis of *N*-(3-bromo-4-fluorophenyl)-4-((1,1-dioxotetrahydro-2*H*-thian-4-yl-2,2,6,6-$d_4$)amino)-*N*'-hydroxy-1,2,5-oxadiazole-3-carboxamidine (Compound 1)**

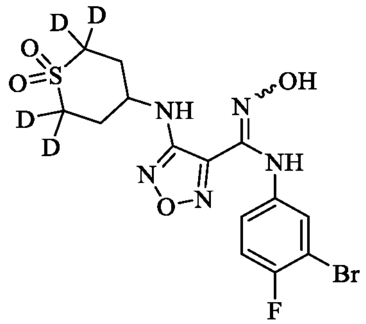

Steps:

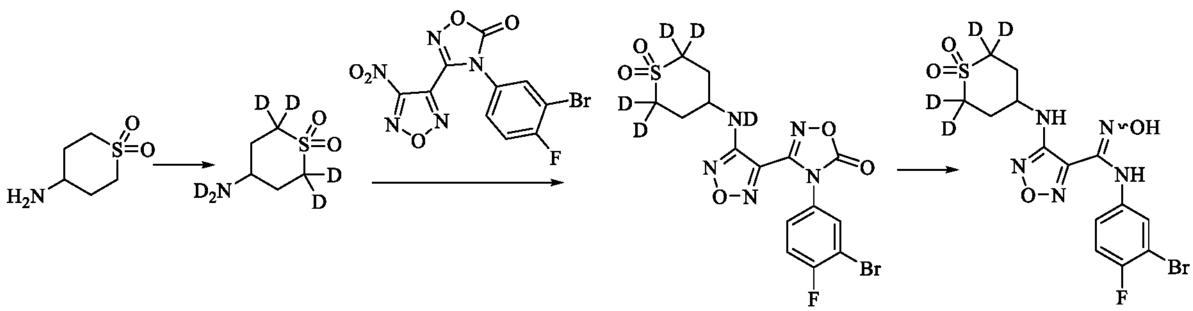

Step 1: Synthesis of 4-(amino-$d_2$)-tetrahydro-2*H*-thian-1,1-dioxide-2,2,6,6-$d_4$

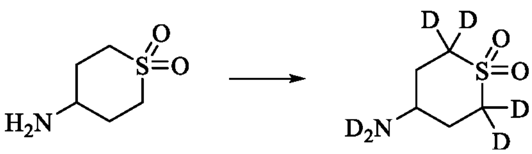

4-Aminotetrahydro-2*H*-thian-1,1-dioxide (800.0 mg, 5.36 mmol, 1.0 eq) was dissolved in $D_2O$ (16 mL), to which was added sodium hydroxide (1.072 g, 26.8 mmol, 5.0 eq), stirred at room temperature for 66 hours, concentrated under reduced pressure, and then $D_2O$ (16 mL) was added to the crude product and stirred at room temperature for 24 hours, when no starting material remained by LC-MS monitoring, concentration under reduced pressure was carried out to obtain the target compound (1.5 g, crude product).

Step 2: Synthesis of 4-(3-bromo-4-fluorophenyl)-3- (4-((1,1-dioxotetrahydro-2*H*-thian-4-yl - 2,2,6,6-$d_4$)amino-*d*)-1,2,5-oxadiazol-3-yl)-1,2,4-oxadiazol-5(4*H*)-one:

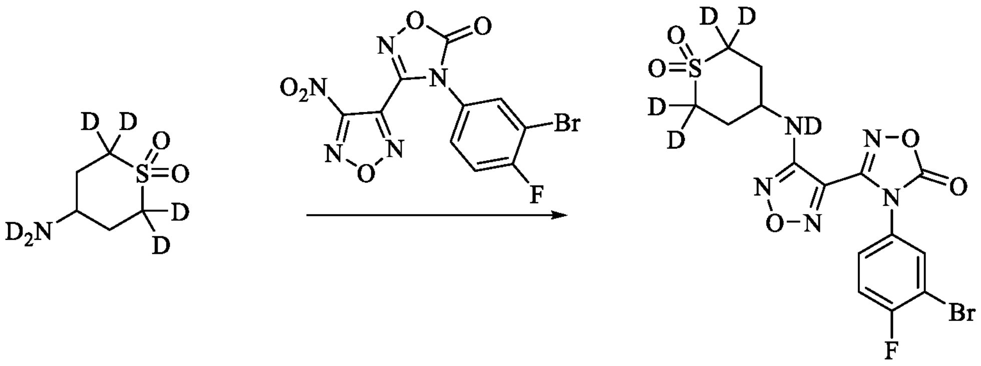

The intermediate 4-(amino-$d_2$)tetrahydro-2*H*-thian-1,1-dioxide-2,2,6,6-$d_4$ (821.3 mg) was dissolved in THF (4 mL) and DMA (4 mL), to which was added DIEA (2.07 g), subjected to ice-cool to 0°C, and a solution of 4-(3-bromo-4-fluorophenyl)-3-(4-nitro- 1,2,5-oxadiazol-3-yl)-1,2,4-oxadiazol-5(4*H*)-one (1.99 g) in tetrahydrofuran (4 mL) was dropwise added with, slowly heated to room temperature and reacted overnight. The reaction solution was concentrated under reduced pressure to obtain the target compound (2.3 g, crude product).

Step 3: Synthesis of *N*-(3-bromo-4-fluorophenyl)-4-((1,1-dioxotetrahydro-2*H*-thian-4-yl-2,2,6,6-$d_4$)amino)-*N*-hydroxy-1,2,5-oxadiazole-3-carboxamidine:

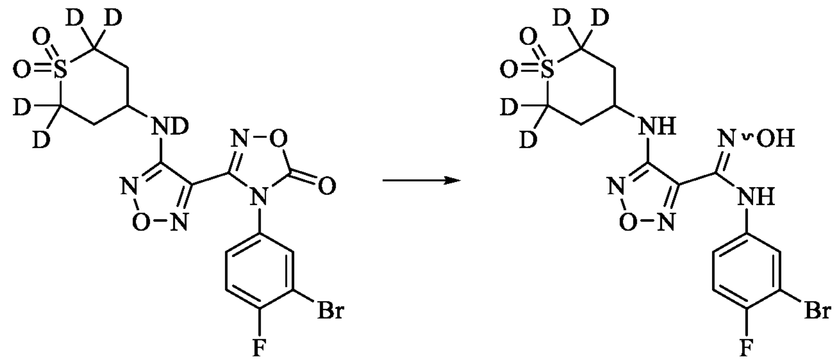

The intermediate 4-(3-bromo-4-fluorophenyl)-3-(4-((1,1-dioxotetrahydro-2*H*-thian-4-yl-2,2,6,6-$d_4$)amino-*d*)-1,2,5-oxadiazol-3-yl)-1,2,4-oxadiazol-5(4*H*)-one (2.56 g) was dissolved in THF (15 mL) and $D_2O$ (4 mL), to which was added sodium hydroxide (320.0 mg), and stirred at room temperature for 1 hour. Water (4 mL) and ethyl acetate (10 mL) were added and the layers were separated. The aqueous phase was extracted with ethyl acetate (3 × 10 mL). The organic phases were combined, washed with water (3 × 4 mL), washed with saturated brine, dried over anhydrous sodium sulfate, filtered, and concentrated. The crude product was purified by silica gel column chromatography (petroleum ether: ethyl acetate = 5: 1 ~ 1: 1) to obtain Compound 1 (59.0 mg) with a deuteration rate of 95%.

$^1$HNMR (400MHz, $CDCl_3$) δ(ppm): 7.82 (s, 1H), 7.27-7.25 (t, 1H), 7.08-7.04 (t, 1H), 6.98-6.94 (m, 1H), 6.01-5.99 (d, 1H), 3.91-3.83 (m, 1H), 2.53-2.48 (m, 2H), 2.41-2.35 (m, 2H).

Molecular formula: $C_{14}H_{11}D_4BrFN_5O_4S$; Molecular weight: 452.29; LC-MS (Pos, *m/z*) = 452.0, 454 (isotope) [M+H]+.

**Example 2: Synthesis of *N*-(3-bromo-4-fluorophenyl)-4-((1,1-dioxotetrahydro-thiophen-3-yl -2,2,5,5-$d_4$)amino)-*N*'-hydroxy-1,2,5-oxadiazole-3-carboxamidine (Compound 2)**

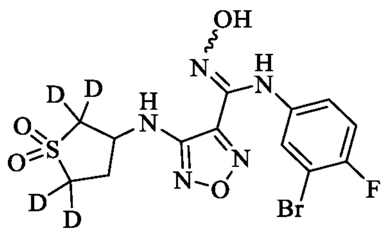

Steps:

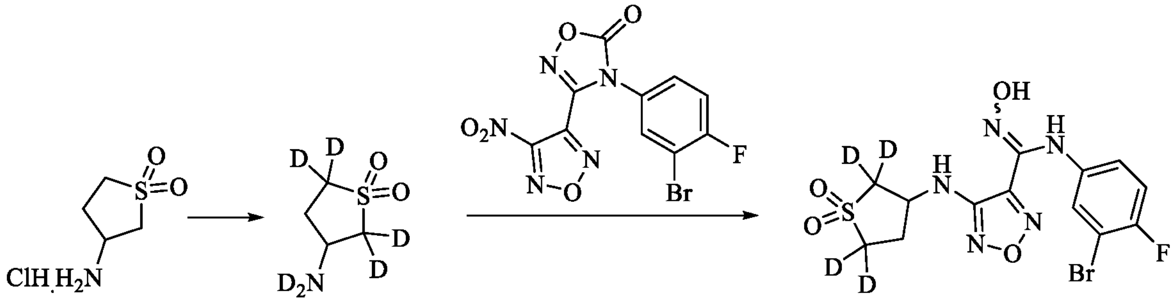

Step 1: Synthesis of 3-(amino-$d_2$)tetrahydrothiophene-1,1-dioxide-2,2,5,5-$d_4$:

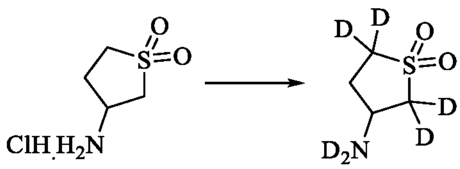

3-Aminotetrahydrothiophene-1,1-dioxide hydrochloride (300.0 mg, 1.75 mmol) was dissolved in $D_2O$ (4.0 mL), to which was added sodium hydroxide (350.0 mg, 8.75 mmol), and stirred at room temperature overnight. When $^1$HNMR showed the deuteration was not completed, concentration was carried out under reduced pressure, $D_2O$ (4.0 mL) and sodium hydroxide (350.0 mg, 8.75 mmol) were added to the crude product and stirred at room temperature overnight. NMR showed the deuteration was not completed, concentration was carried out under reduced pressure, $D_2O$ (4.0 mL) was added to the crude product and stirred at room temperature overnight, $^1$HNMR showed the deuteration rate was about 98.1%, and concentration was carried out under reduced pressure to give 3-(amino-$d_2$)tetrahydrothiophene-1,1-dioxide 2,2,5,5-d4 (280.0 mg, crude product).

Step 2: Synthesis of *N*-(3-bromo-4-fluorophenyl)-4-((1,1-dioxotetrahydro-thiophen-3-yl - 2,2,5,5-$d_4$)amino)-*N'*-hydroxy-1,2,5-oxadiazole-3-carboxamidine:

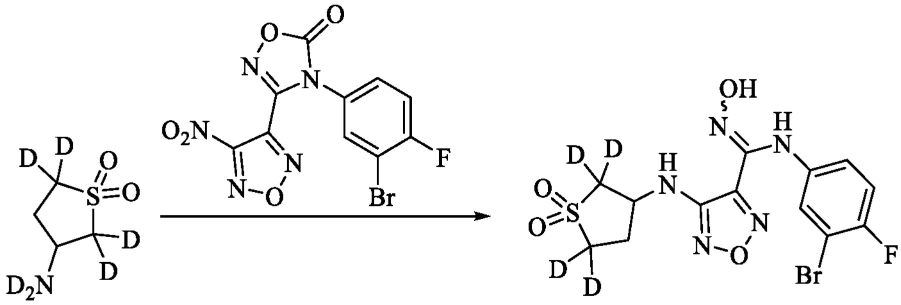

The intermediate 4-(3-bromo-4-fluorophenyl)-3-(4-nitro-1,2,5-oxadiazol-3-yl)-1,2,4-oxadiazol-5(4*H*)one (647.3 mg, 1.74 mmol) and 3-(amino-$d_2$)tetrahydrothiophene-1,1-dioxide-2,2,5,5-d4 (242.30 mg, crude product) were dissolved in water (3.5 mL) and tetrahydrofuran (4 mL), to which was added sodium carbonate (601.2 mg, 4.35 mmol), stirred at room temperature overnight. TLC detection showed the reaction was completed, and NaOH (1 mol/L, 4 mL) was added, and stirred at room temperature for 2 hours. When TLC detection showed the reaction was completed, water (4 mL) and ethyl acetate (10 mL) were added, and the layers were separated. The aqueous phase was extracted with ethyl acetate (3 × 10 mL), the organic phases were combined, washed with water (3 × 4 mL), washed with saturated brine, dried over anhydrous sodium sulfate, filtered, concentrated, and the crude product was subjected to silica gel column chromatography (PE: EA = 4:1, 3:1, 2:1, 1:1) purification to obtain *N*-(3-bromo-4-fluorophenyl)-4-((1,1-dioxotetrahydro-thiophen-3-yl-2,2,5,5-$d_4$)amino)-*N*'-hydroxy-1,2,5-oxadiazole-3-carboxamidine (200.0 mg, yield 26.3%).

$^1$HNMR (400MHz, DMSO-$d_6$) δ(ppm): 11.47 (s, 1H), 8.92 (s, 1H), 7.20-7.12 (m, 1H), 7.11-7.10 (d, 1H), 6.78-6.75 (m , 1H), 6.64-6.62 (d, 1H), 4.32-4.27 (m, 1H), 2.46-2.45 (d, 1H), 2.21-2.16 (m, 1H).

Molecular formula: $C_{13}H_9D_4BrFN_5O_4S$; Molecular weight: 438.26; LC-MS (Pos, *m*/*z*) = 438.02 $[M+H]^+$

**Example 3: Synthesis of *N*-(3-bromo-4-fluorophenyl)-4-((1,1-dioxothiacyclobutan-3-yl-2,2,4,4-$d_4$)amino)-*N*'-hydroxy-1,2,5-oxadiazole-3-carboxamidine**

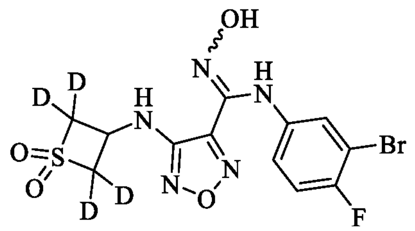

Steps:

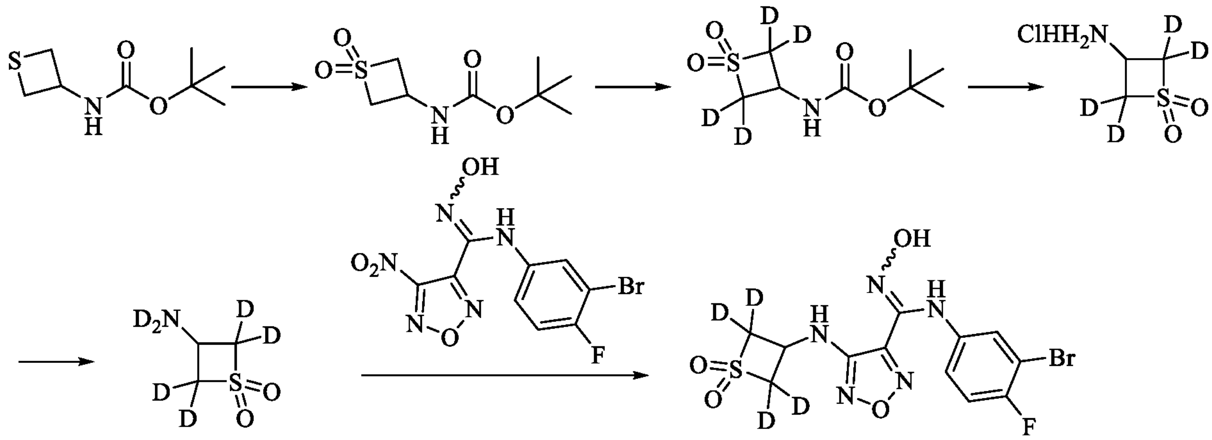

Step 1: Synthesis of tert-butyl (1,1-dioxothiacyclobutan-3-yl)carbamate:

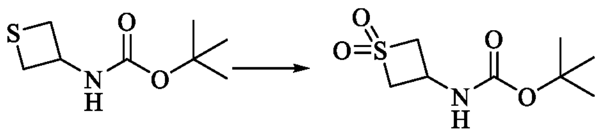

Tert-butyl thiacyclobutan-3-ylcarbamate (300.0 mg, 1.59 mmol) was dissolved in dichloromethane (10 mL), to which was added m-chloroperoxybenzoic acid (mass fraction 70%, 980.0 mg, 3.98 mmol), reacted under stirring overnight at room temperature. When TLC monitored the completion of the reaction, a saturated sodium bicarbonate aqueous solution

(10 mL) was added, and the layers were separated. The aqueous phase was extracted with DCM (3 × 15 mL). The organic phases were combined, dried over anhydrous magnesium sulfate, filtered, and concentrated. The crude product was passed through a silica gel column chromatography (DCM: MeOH = 100:1 ~ 40:1) and purified to give tert-butyl (1,1-dioxothiacyclobutan-3-yl)carbamate as a white solid (300.0 mg, yield: 89.6%).

Step 2: Synthesis of tert-butyl (1,1-dioxothiacyclobutan-3-yl-2,2,4,4-*d*$_4$)carbamate

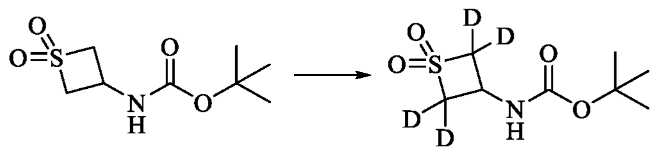

Tert-butyl (1,1-dioxothiacyclobutan-3-yl)carbamate (300.0 mg, 1.36 mmol) was dissolved in deuteroxide (4 mL) and tetrahydrofuran (4 mL), to which was added sodium hydroxide (544.0 mg, 13.6 mmol), stirred at room temperature overnight, deuteroxide (2 mL) was added, extracted with EA (3 × 10 mL); the organic phases were combined, dried over anhydrous sodium sulfate, and concentrated under reduced pressure. The above operation was repeated twice more to obtain tert-butyl (1,1-dioxothiacyclobutan-3-yl-2,2,4,4-*d*$_4$)carbamate (220.0 mg, crude product), NMR detection showed the deuteration was not completed, and the product was not purified and used directly in the next step.

Step 3: Synthesis of 3-aminothiacyclobutane 1,1-dioxide-2,2,4,4-*d*$_4$ hydrochloride

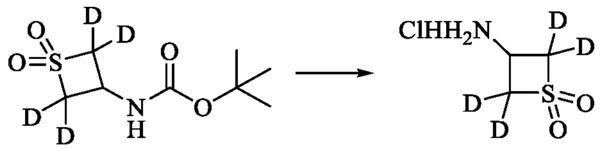

Tert-butyl (1,1-dioxothiacyclobutan-3-yl-2,2,4,4-*d*$_4$)carbamate (220.0 mg, crude product) was dissolved in dichloromethane (5 mL), cooled with ice bath to 0°C, hydrogen chloride ethanol solution (3 mL) was added, slowly elevated the temperature to room temperature, and reacted for 2 hours. When TLC detection showed the reaction was completed, concentration was carried out under reduced pressure to give 3-aminothiacyclobutane 1,1-dioxide-2,2,4,4-*d*$_4$ hydrochloride (170.0 mg crude).

Step 4: Synthesis of 3-(amino-*d*$_2$)thiacyclobutane-1,1-dioxide-2,2,4,4-*d*$_4$

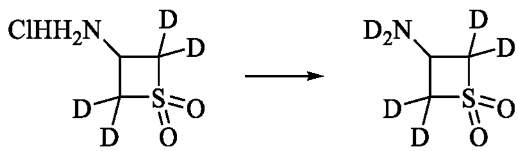

3-Aminothiacyclobutane-1,1-dioxide-2,2,4,4-*d*$_4$ hydrochloride (158.3 mg) was dissolved in $D_2O$ (5 mL), to which was added calcium oxide (165.0 mg, 2.94 mmol), reacted at room temperature overnight. After suction filtration, the filtrate was concentrated under reduced pressure to obtain 3-(amino-*d*$_2$)thiacyclobutane-1,1-dioxide-2,2,4,4-*d*$_4$ (125.1 mg, crude product).

Step 5: *N*-(3-bromo-4-fluorophenyl)-4-((1,1-dioxothiacyclobutan-3-yl-2,2,4,4-*d*$_4$)amino)-*N*'-hydroxy-1,2,5-oxadiazole-3-carboxamidine

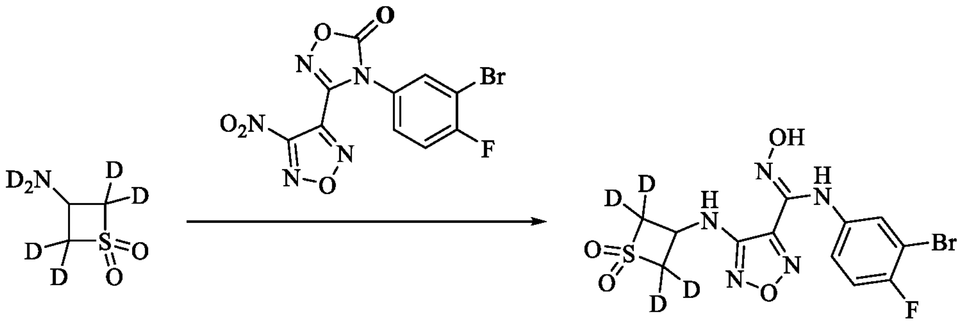

4-(3-Bromo-4-fluorophenyl)-3-(4-nitro-1,2,5-oxadiazol-3-yl)-1,2,4 -oxadiazol-5(4*H*)-one (238.10 mg, 0.64 mmol) and 3-(amino-$d_2$)thiacyclobutane 1,1-dioxide-2,2,4,4-$d_4$ (80.0 mg, crude product) were dissolved in THF (4 mL) and DMF (2 mL), to which was added DIEA (249.41 mg, 1.92 mmol), stirred at room temperature overnight. When TLC detection showed the reaction was completed, NaOH (1 mol/L, 3 mL) was added and stirred at room temperature for 2 hours. When TLC detection showed the reaction was completed, water (4 mL) and ethyl acetate (10 mL) were added, the layers were separated, the aqueous phase was extracted with ethyl acetate (3 × 10 mL), the organic phases were combined, washed with water (3 × 4 mL), washed with saturated brine, dried over anhydrous sodium sulfate, filtered, concentrated, and the crude product was separated by preparative thin layer chromatography (DCM:MeOH = 20:1) to give *N*-(3-bromo-4-fluorophenyl)-4-((1,1-dioxothiacyclobutan-3-yl-2,2,4,4-$d_4$)amino)-*N'*-hydroxy-1,2,5-oxadiazole-3-carboxamidine (20.0 mg, yield: 7.4%).

$^1$HNMR (400MHz, DMSO-$d_6$) δ(ppm): 11.55 (s, 1H), 8.94 (s, 1H), 7.19-7.13 (m, 1H), 7.12-7.11 (t, 1H), 6.96-6.95 (d , 1H), 6.78-6.74 (m, 1H), 4.29-4.28 (d, 1H).

Molecular formula: $C_{12}H_7D_4BrFN_5O_4S$; Molecular weight: 424.24; LC-MS (Neg, *m/z*) = 422.05 $[M-H]^-$

**Example 4: Preparation of Crystalline Form A of Compound 1**

The Compound 1: 4-(3-bromo-4-fluorophenyl)-3-(4-((1,1-dioxotetrahydro-2*H*-thiapyran-4-yl-2,2,6,6-$d_4$)amino-*d*)-1,2,5-oxadiazol-3-yl)-1,2,4-oxadiazol-5(4*H*)-one (225 g)prepared in the Step 3 of Example 1 or of Example 5 was dissolved in THF (1.5 L), to which was added a solution of sodium hydroxide (56.45 g) in water (1 L), and stirred at room temperature for 1 hour. Ethyl acetate (1 L) was added, and the layers were separated. The aqueous phase was extracted with ethyl acetate (2 × 1 L). The organic phases were combined, washed with saturated brine (300 mL), dried over anhydrous sodium sulfate, filtered, and concentrated to obtain a crude product. The crude product was added to anhydrous methanol and slurried at 15°C for 15 hours, subjected to suction filtration, and the filter cake was rinsed with methanol to obtain a pale pink solid, which was dried under vacuum at 30°C for 21 hours to obtain 181.03g of Crystalline Form A with a yield of 85%.

Using Cu-Kα radiation, the X-ray powder diffraction of Crystalline Form A showed characteristic peaks at 8.8±0.2°, 18.0±0.2°, 19.5±0.2°, 22.4±0.2°, 23.1±0.2°, 23.8±0.2°, 27.8±0.2°, and further characteristic peaks at 11.9±0.2°, 17.6±0.2°, 18.7±0.2°, 25.1±0.2°, 26.8±0.2°, and 30.0±0.2°, expressed with 2θ angles (°). As shown in Figure 1.

The melting temperature of Crystalline Form A measured by differential scanning calorimeter was about 219 ~ 220 °C. As shown in Figure 2.

**Example 5: Preparation of Crystalline Form B of Compound 1**

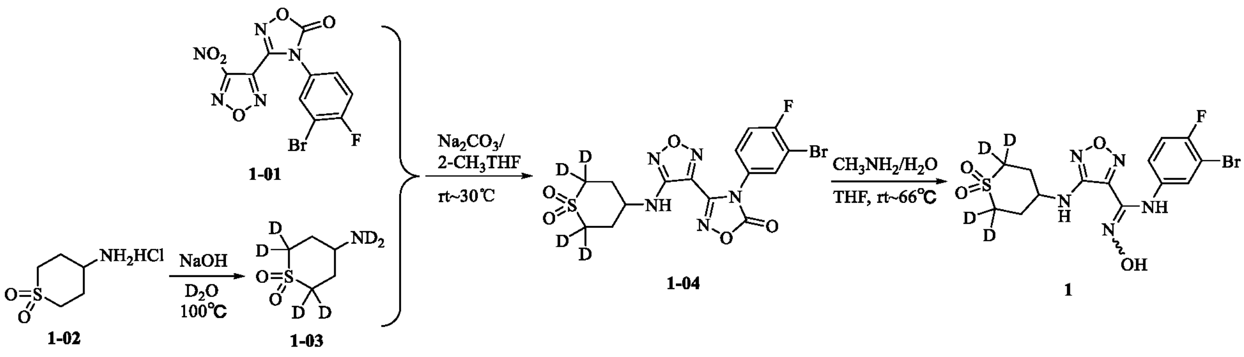


Step 1: Preparation of 1-03

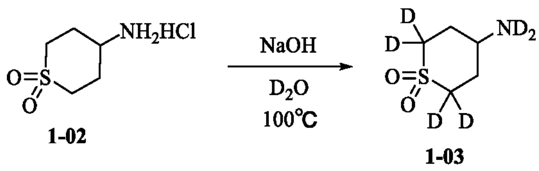


Under nitrogen protection, 1-02 (500 g, 2.693 mol, 1.0 eq) and sodium hydroxide (161.58 g, 4.039 mol, 1.5 eq) were added to deuteroxide (500 g) in a 3L single-necked flask, heated at reflux for 3 hours. After the heating was stopped, concentration was carried out under reduced pressure to obtain an off-white solid, deuteroxide (500 g) was added again, and heated at reflux for 3 hours. The deuteration operation was repeated for 5 times. The deuteration rate measured by NMR was as follows:

| | Deuterated once | | | Deuterated twice | Deuterated 3 times | Deuterated 4 times | Deuterated 5 times |
|---|---|---|---|---|---|---|---|
| Loading amount | Alkali | Equivalen t | Deuteratio n rate | Deuteration rate | Deuteration rate | Deuteration rate | Deuteration rate |
| 500 | NaO H | 1.5 | 69.12% | 88% | 94.8% | 97.9% | 99% |

The reaction system at the last deuteration was not treatmed and used directly in the next step.

Step 2: Preparation of 1-04

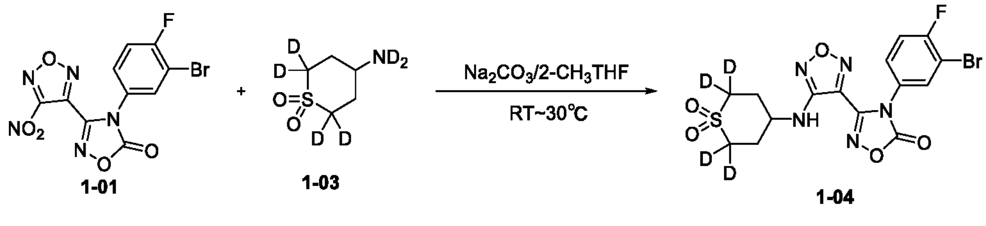


Under nitrogen protection, to a 10L four-neck flask, a solution of 1-03 (5.385mol, 1.6eq) in deuteroxide (pH was 13-14) was added, and a solution of 1-01 (1250g, 3.36mol, 1.0 eq) prepared in Step 1 for several times in 2-methyltetrahydrofuran (3.5 L) was dropwise added. After the addition, the reaction was carried out for 2 hours, and the pH was measured at 8-9. Anhydrous sodium carbonate (284.86 g, 2.688 mol, 0.8 eq) was added, the temperature was gradually elevated to 30°C and the reaction was carried out for 16 hours. After the completion of the reaction, suction filtration was carried out, the filter cake was slurried and washed with 4L of water for 2 hours, slurried and washed with 4L of acid aqueous

solution (which pH was adjusted with 2mol/L diluted hydrochloric acid, and maintained pH = 3-4) for 1 hour. After suction filtration, the filter cake was slurried and washed with 2.5L of anhydrous methanol for 16 hours. After filtration, the filter cake was rinsed with 500 mL of anhydrous methanol and dried with forced air at 40°C for 24 hours to give 1386.2 g of off-white solid with a yield of 86.3%.

Step 3: Preparation of Compound 1

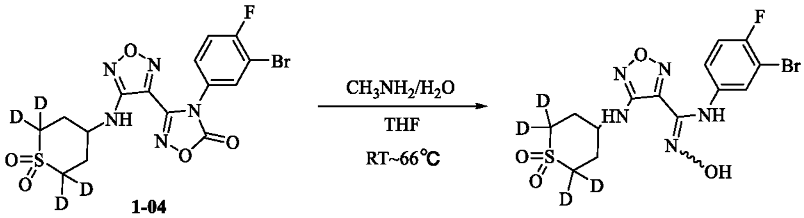


1-04

1

Under nitrogen protection, to a 5L four-necked flask, a suspension of 1-04 (1150g, 2.404 mol) in THF (2.3L) was added, and methylamine aqueous solution (mass fraction 25% to 30%, 896.17g, 7.213 mol, 3.0 eq) was added. The temperature was slowly raised to reflux (about 66°C), sampling was carried out after 1.5 hours and HPLC showed that the reaction was completed. A small amount of diatomite was spread, and suction filtration was carried out, the filter cake was rinsed with 2.5L of ethyl acetate, and to the filtrate was added 1L of 10% brine, the layers were separated, the aqueous phase was extracted with ethyl acetate (1L), the organic phases were combined, a mixed solution of concentrated hydrochloric acid (200 ml, 2.4 mol, 1 eq) and 10% saline (800 ml) were added, washed once, the organic phase was subjected to suction filtration and concentrated under reduced pressure to obtain Compound 1.

Step 4: Preparation of Crystalline Form B

The Compound 1 obtained in Step 3 was added in 10 L of acetone, refluxed for 1 hour, and subjected to suction filtration. When the filtrate was distilled to about 5 L under normal pressure, a solid gradually precipitated in the system. The distillation was unceasingly carried out to about 3 L under normal pressure, and 5 L of ethyl acetate was dropwise added. After the dropwise addition, when the distillation was carried out at normal pressure to about 3L, the heating was stopped, the system was slowly cooled to room temperature and slurried for 17 hours; after suction filtration, the filter cake was rinsed with 1L of ethyl acetate, and dried under vacuum at 40°C for 4 hour to obtain 877.0g of Crystalline Form B (HPLC: 99.62%) with a yield of 80.6%.

$^1$HNMR (400 MHz, DMSO-$d_6$) δ(ppm): 11.42 (s, 1H), 8.91 (s, 1H), 7.20-7.15 (m, 1H), 7.12-7.09 (m, 1H), 6.81-6.76 (m, 1H), 6.38 (d, J = 7.2 Hz, 1H), 3.73-3.71 (m, 1H), 2.23-2.19 (m, 2H), 2.06-2.00 (m, 2H).

Molecular formula: $C_{14}H_{11}D_4BrFN_5O_4S$; Molecular weight: 452.29; LC-MS (Neg, *m/z*) = 450.0, 452.0 (isotope) $[M-H]^-$

Using Cu-Kα radiation, the X-ray powder diffraction of Crystalline Form B showed characteristic peaks at 16.2±0.2°, 18.0±0.2°, 21.1±0.2°, 21.6±0.2°, 21.8±0.2°, 23.7± 0.2°, 23.9±0.2°, 26.2±0.2°, and further characteristic peaks at 10.7±0.2°, 15.8±0.2°, 16.7±0.2°, 19.3±0.2°, 20.1±0.2°, 22.5±0.2°, 23.2±0.2°expressed with 2θ angles (°). As shown in Figure 3.

The melting temperature of Crystalline Form B measured by differential scanning calorimeter was about 236 ~ 238°C. As shown in Figure 4.

As shown in Figure 5, according to the integrated area, the deuteration rate of the product as calculated was 99.51%, and the purity of the Compound 1 was not less than 98%.

**Example 6: Synthesis of 4-amino-*N*-(3-bromo-4-fluorophenyl)-*N'*-hydroxy-1,2,5-oxadiazole-3-carboxamidine**

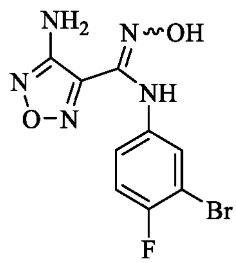

Step 1: Synthesis of 4-amino-*N*'-hydroxy-1,2,5-oxadiazole-3-carboxamidine

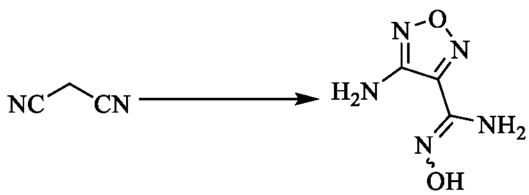

Malononitrile (50.0 g, 0.76 mol, 1.0 eq) as raw material was added into a four-necked flask, heated to dissolve (about 50 °C), water (0.5 L) was added, subjected to ice water bath at about 10 °C, and added in batches of sodium nitrite (57.72 g, 0.83 mol, 1.1 eq). After the addition, hydrochloric acid (6N, 8.5 mL) was added dropwise below 10°C. After the dropwise addition, the reaction solution was stirred for 0.5 hours in an ice-water bath until the temperature remained constant. The reaction solution was referred to as A. $H_2NOH{\cdot}HCl$ (158.4 g, 2.28 mol, 3.0 eq) was dissolved in water (255 mL), a solution of potassium hydroxide (127.9 g, 2.28 mol, 3.0 eq) in water (255 mL) was added, and then stirred at room temperature (25°C) for 10 minutes, and the above reaction solution was referred to as B. The reaction solution A was cooled to 0°C to 10°C with an ice water bath, and the reaction solution B was added dropwise to the reaction solution A. After the dropwise addition, agitation was carried out under ice water bath for 0.5 hours until the temperature remained constant. The ice water bath was removed and the reaction solution was heated and refluxed for 12 hours. After the reaction was completed, hydrochloric acid (6N, 120mL) was added dropwise under an ice-water bath (0°C) to adjust the pH = 7, and the agitation was continued for 40 minutes. The reaction solution was subjected to suction filtration, the filter cake was washed with water, and the filter cake was dried to obtain the target compound (101g, yield: 93.5%).

Step 2: Synthesis of 4-amino-*N*-hydroxy-1,2,5-oxadiazole-3-methylenimidoyl chloride

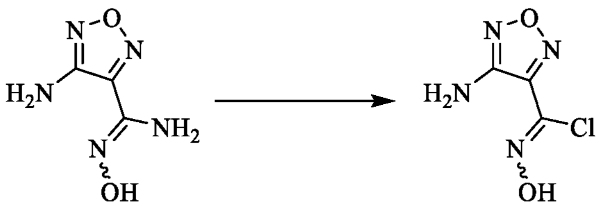

To a reaction flask, 4-Amino-*N*'-hydroxy-1,2,5-oxadiazole-3-carboxamidine (101g, 0.71mol, 1.0eq) was added, water (1.4L) was added and stirred, HCl (6N, 350mL) and AcOH (710mL) were added under stirring, heated (42°C to 45°C) and stirred until the solution was clear, NaCl (124.5g, 2.13mol, 3.0eq) was added, and a solution of $NaNO_2$ (48.3g, 0.70 mol, 0.98 eq) in water (168 mL) was added dropwise under stirring in an ice-water bath, and the dropwise adding was finished within 3.5 hours; after the addition, agitation was carried out for 1.5 hours in an ice-water bath, the temperature was slowly elevated to room temperature (25°C) and agitation was carried out for about 1 hour. The reaction solution was subjected to suction filtration, the filter cake was washed with water, and the filter cake was dried to obtain 4-amino-*N*-hydroxy-1,2,5-oxadiazole-3-methylenimidoyl chloride (crude product, 36.78 g, yield: 32%).

Step 3: Synthesis of 4-amino-*N*-(3-bromo-4-fluorophenyl)-*N*'-hydroxy-1,2,5-oxadiazole-3-carboxamidine

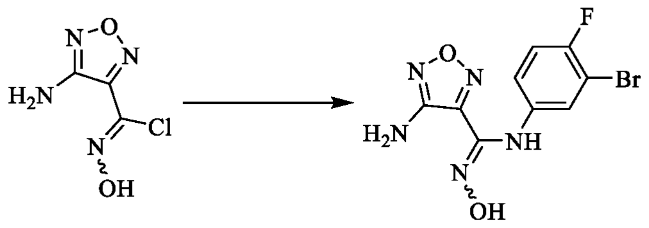

4-amino-*N*-hydroxy-1,2,5-oxadiazole-3-methylenimidoyl chloride (36.78 g, 0.23 mol, 1.0 eq) was dissolved in water (318 ml), heated under stirring to 60°C, 3-bromo-4-fluoroaniline (47.50g, 0.25 mol, 1.1eq) was added, stirred at 60°C for about 10 minutes, a solution of $NaHCO_3$ (29.40g, 0.35 mmol, 1.5eq) in water (318mL) was dropwise added, and the dropwise addition was finished within 20 minutes. After the dropwise addition, the reaction solution was stirred at 60°C for 30 minutes, and cooled to room temperature. The reaction solution was subjected to suction filtration, the filter cake was washed with water, the filter cake was suction dried and then slurried with water overnight, subjected to suction filtration on the next day to obtain a gray solid, which was dried to obtain the target product (61.21g, yield: 84.2%).
$^1$HNMR (400MHz, $d_6$-DMSO) $\delta$(ppm): 11.44 (s, 1H), 8.88 (s, 1H), 7.17-7.21 (t, 1H), 7.09-7.12 (d, 1H), 6.75-6.79 (m, 1H), 6.26 (s, 2H).
Molecular formula: $C_9H_7BrFN_5O_2$; Molecular weight: 316.09; LC-MS (Neg, *m*/*z*) = 314.0 $[M-H]^-$.

**Example 7: Synthesis of 4-(3-bromo-4-fluorophenyl)-3-(4-nitro-1,2,5-oxadiazol-3-yl)- 1,2,4-oxadiazol-5(4*H*)-one**

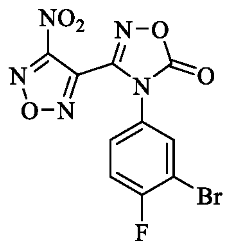

Step 1: Synthesis of 3-(4-amino-1,2,5-oxadiazol-3-yl)-4-(3-bromo-4-fluorophenyl)-1,2,4-oxadiazole-5(4*H*)-one

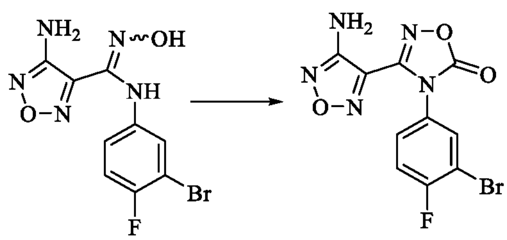

4-Amino-*N*-(3-bromo-4-fluorophenyl)-*N*'-hydroxy-1,2,5-oxadiazole-3-carboxamidine (5.0 g, 15.8 mmol, 1.0 eq) was dissolved in ethyl acetate (65 mL), carbonyldiimidazole (3.85 g, 23.7 mmol, 1.5 eq) was added, heated and reacted at 60°C for 0.5 hours. After TLC detection showed the reaction was completed, the reaction solution was cooled to room temperature and washed with 1 mol/L hydrochloric acid (65 mL × 2), the organic phases were combined, the separated organic phases was concentrated and dried, the crude product was slurried with methyl tert-butyl ether, filtered and dried to obtain 3-(4-amino-1,2,5-oxadiazol-3-yl)-4- (3-bromo-4-fluorophenyl)-1,2,4-oxadiazol-5(4*H*)-one (3.53 g, yield: 65%).

Step 2: Synthesis of 4-(3-bromo-4-fluorophenyl)-3-(4-nitro-1,2,5-oxadiazol-3-yl)- 1,2,4-oxadiazol-5(4*H*)-one

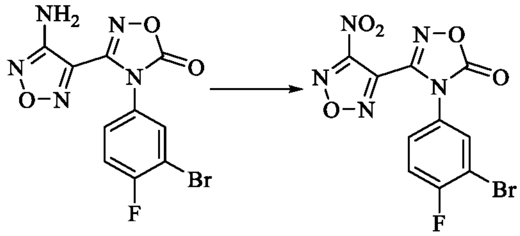

3-(4-Amino-1,2,5-oxadiazol-3-yl)-4-(3-bromo-4-fluorophenyl)-1,2,4-oxadiazol-5(4*H*)-one (264mg, 0.8mmol, 1.0 eq) was dissolved in trifluoroacetic acid (5 mL), hydrogen peroxide (3 mL, 30%) was added, and reacted at 45°C for 18 hours. When TLC detection showed the reaction was completed, the reaction solution was cooled to room temperature and saturated sodium bicarbonate solution was added in ice bath until the solution did not bubble, then extracted by adding EA (3 × 30 mL), dried over anhydrous sodium sulfate, and subjected to suction filtration, the filtrated was concentrated, and the crude product was subjected to silica gel column chromatography (PE:EA = 10:1 to 2:1) to give 4-(3-bromo-4-fluorophenyl)-3- (4-nitro-1,2,5-oxadiazol-3-yl)-1,2,4-oxadiazol-5(4*H*)-one (189.9mg, yield: 66%).
$^1$HNMR (400MHz, DMSO-d6): 8.06-8.04 (m, 1H), 7.69-7.65 (m, 1H), 7.60-7.55 (m, 1H).

**Example 8: Synthesis of *N*-(3-bromo-4-fluorophenyl)-4-((1,1-dioxotetrahydro-2*H*-thian-4-yl) amino)-*N*'-hydroxy-1,2,5-oxadiazole-3-carboxamidine (Compound R)**

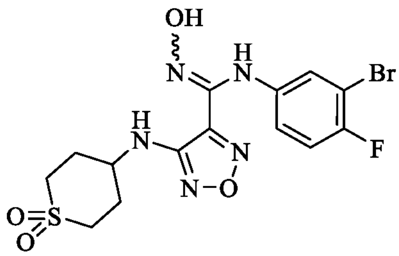

Compound R

Step 1: Synthesis of 4-(3-bromo-4-fluorophenyl)-3-(4-((1,1-dioxotetrahydro-2*H*-thian-4-yl) amino)-1,2,5-oxadiazol-3-yl)-1,2,4-oxadiazol-5(4*H*)-one

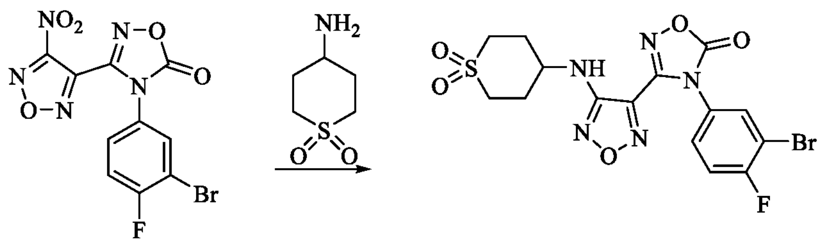

4-(3-Bromo-4-fluorophenyl)-3-(4-nitro-1,2,5-oxadiazol-3-yl)-1,2,4-oxadiazol-5(4*H*)-one (250.4 mg, 0.67 mmol, 1.0 eq) was dissolved in DMA (4 mL), 4-aminotetrahydro-2*H*-thiane 1,1-dioxide (200.8 mg, 1.35 mmol, 2.0 eq) was added in ice bath, stirred and reacted for 1 hour in an ice bath. After TLC detection showed the reaction was completed, water (30 mL) was added, extraction was carried with EA (3 × 30 mL), the organic phases were combined, dried over anhydrous magnesium sulfate, filtrated, concentrated, and the crude product was passed through the column chromatography (PE:EA = 10:1 to 1:1) to obtain 4-(3-bromo-4-fluorophenyl)-3-(4-((1,1-dioxotetrahydro-2*H*-thian-4-yl)amino)-1,2,5-oxadiazol-3-yl)-1,2,4-oxadiazol-5(4*H*)-one (78.9 mg, yield: 25%) .

Step 2: Synthesis of N-(3-bromo-4-fluorophenyl)-4-((1,1-dioxotetrahydro-2*H*-thian-4-yl) amino)-*N*'-hydroxy-1,2,5-oxadiazole-3-carboxamidine

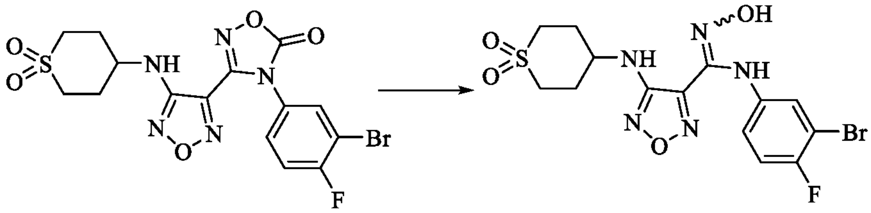

4-(3-Bromo-4-fluorophenyl)-3-(4-((1,1-dioxotetrahydro-2*H*-thian-4-yl)amino)-1,2,5-oxadiazol-3-yl)-1,2,4-oxadiazol-5(4*H*)-one (78.9 mg, 0.17 mmol, 1.0 eq) was dissolved in THF (10 mL), 2 mol/L (2 mL) sodium hydroxide solution was added, stirred and reacted for 30 minutes. After TLC detection showed the reaction was completed, 1 mol/L hydrochloric acid solution was used to adjust the pH to 2 to3; extraction was carried out with EA (3 × 20 mL), the organic phases were combined, dried over anhydrous magnesium sulfate, filtered, and concentrated, the crude product was subjected to silica gel column chromatography (PE:EA = 10:1 to 1:1) to obtain N-(3-bromo-4-fluorophenyl)-4-((1,1-dioxotetrahydro-2*H*-thian-4-yl)amino)-*N*'-hydroxy-1 ,2,5-oxadiazole-3-carboxamidine (26 mg, yield: 35%).
$^1$HNMR (400MHz, DMSO-$d_6$) $\delta$(ppm): 11.42 (s, 1H), 8.91 (s, 1H), 7.18 (m, 1H), 7.09-7.10 (m, 1H), 6.77-6.79 (m, 1H), 6.37-6.39 (m, 1H), 3.70-3.72 (m, 1H), 3.23-3.29 (m, 2H), 3.06-3.09 (m, 2H), 2.20-2.23 (m, 3H), 2.02-2.05 (m, 3H).

Molecular formula: $C_{14}H_{15}BrFN_5O_4S$; Molecular weight: 448.27; LC-MS (*m/z*) = 448.0 $[M+H]^+$

The present invention can be better understood from the following biological test examples. However, those skilled in the art can easily understand that the content described in the test examples is only used to illustrate the present invention, and should not be limited to the present invention.

**Biological Test exampleTest examples**

**Test exampleTest example 1: Enzymatic evaluation methodology**

Test substance: Compound 1 of the present invention, prepared according to the method of Example 1 or the Step 3 of Example 5

I. Experimental materials and instruments

IDO-1, TDO (In House)

Catalase (purchased from Sigma, Cat. No. C30)

L-Tryptophan (purchased from Sigma, Cat. No. 93659-10G)

L-ascorbic acid (purchased from Sigma, Cat. No. A5960)

Methylene blue hydrate (purchased from Sigma, Cat. No. 66720)

DMSO (purchased from Sigma, Cat. No. D2650)

96-well cell culture plate (Corning, Cat. No. 3603)

II. Experimental steps

Preparation of compound: The compound was made up into 1 mM compound mother liquor with DMSO, and then the compound was diluted 3.16 times with DMSO to obtain 10 concentration gradients (100 ×) of the compound

of the diluted mother liquors..

Medication and incubation: 10 μL of 100× compound of the diluted mother liquors were added into a 96-well plate, to which was added 10 μL of enzyme solution (IDO-1 or TDO), centrifuged at 2000 rpm for 1 minute, and incubated at room temperature for 30 minutes. 4% DMSO enzyme-free solution was used as a negative control, and 4% DMSO enzyme-containing solution was used as a positive control. 20 μL of substrate mixture (L-tryptophan, ascorbate, methylene blue, catalase) was added and incubated for 1 hour at room temperature, then 20 μL of stop solution was added to the experimental plate to terminate the reaction. After shaking and mixing, incubation was carried out at 60 °C for 30 minutes. After centrifugation at 2000 rpm for 5 minutes, 45 μL of the supernatant was taken and added into a fresh experimental plate, 45 μL of chromogenic reagent was added, mixed by shaking, and incubated at room temperature for 15 minutes.

Detection: A microplate reader was used to detect the absorbance value at 492 nm.

Calculation: The inhibition rate was calculated according to the following formula, using the Spectramax program to fit the curve slope, and using GraphPad Prism5.0 to fit the IC50:

$$Inhibition_rate_(\%) = 100\% - \frac{OD_{compound} - OD_{negative_control}}{OD_{positive_control} - OD_{negative_control}} \times 100\%$$

III. Test results

Table 1: Enzymatic assay of the compound of the present invention

| Compound No. | $IC_{50}$ (nM) | |
|---|---|---|
| | IDO | TDO |
| 1 | 85.38 | 5043 |

From the results in Table 1, it could be seen that the compound of the present invention had excellent IDO enzyme inhibitory activity, and showed higher selectivity relative to TDO, indicating that the compound of the present invention was a highly selective IDO inhibitor.

**Test example 2: Cytological evaluation methodology**

Test substances: Compounds 1-3 of the present invention, prepared as described in the previous specific Examples.

I. Experimental materials and instruments

Hela cell line (Cell Bank, Chinese Academy of Sciences)

Recombinant human IFN-γ (rhIFN-γ, purchased from R&D Systems, Cat. No. 285-IF-100)

Phenol red-free DMEM medium (purchased from Gibco, Cat. No. 21063029)

Fetal bovine serum (purchased from Gibco, Cat. No. 10099-141)

Trichloroacetic acid (purchased from Sigma, Cat. No. T9159)

II. Experimental steps

Cell plating: Hela cell suspension was prepared with fresh phenol red-free DMEM medium, and 20,000 cells/well were added to a 96-well cell culture plate, and cultured in 5% carbon dioxide at 37 °C overnight.

Preparation of compounds: the compounds were made up into 0.5 mM solution with DMSO, and the compounds were diluted 3.16 times with DMSO to obtain 8 compound mother liquors of concentration gradients (500×), and diluted with cell culture medium to prepare 10× dilutions.

Medication and incubation: After the cells were seeded and cultured overnight, 10 μL of a compound of the corresponding diluted mother liquor (10×) was added to each well and incubated for 1 hour, then 10 μL of 500ng/ml rhIFN-γ was added, the final volume of each well was 100 μL. The negative control wells contained 100 μL of 0.5% DMSO cell culture medium and Hela cells. The positive control wells were the wells where rhIFN-γ at a final concentration of 50ng/ml was added to the negative control wells, and the background control wells contained 100 μL of cell culture medium. After incubation in a 37 °C incubator for 24 hours. the cell morphology was observed under an inverted microscope.

Detection: After the cell plate was centrifuged, 80 μL of the supernatant was taken and added to a Corning 96-well plate, 10 μL of trichloroacetic acid was added to each well, shaken, placed in a 60 °C incubator for 30 minutes, and centrifuged at 2500 rpm for 5 minutes. 45 μL of the supernatant was taken and transferred to a new plate, 45 μL of chromogenic solution was added, shaken to make the reaction uniform. After incubating at room temperature for 15 minutes, the absorbance at 492 nm wavelength was read.

III. Test results

Table 2: Cytological activity test of the compounds of the present invention

| Compound No. | Hela Cell $IC_{50}$ (nM) |
|---|---|
| 1 | 99.68 |
| 2 | 50.55 |
| 3 | 49.64 |

According to the above experimental method, it was also measured that the Crystalline Form A prepared in Example 4 and the Crystalline Form B prepared in Example 5 of Compound 1 of the present invention showed the cytological $IC_{50}$ as follows:

Table 3: Cytological activity test of the Crystalline Forms of the present invention

| Compound No. | Hela Cell $IC_{50}$ (nM) |
|---|---|
| Crystal Form A of Compound 1 | 40 |
| Crystal Form B of Compound 1 | 30 |

From the above results, it could be seen that both the compounds and their Crystalline Forms of the present invention had excellent cytological activity to Hela cells, thereby having clinical value in inhibiting tumor cell proliferation.

**Test example 3: Evaluation of pharmacokinetics in mice**

Test substance: Compound 1 of the present invention,prepared according to the method of Example 1 or Step 3 of Example 5;
Compound R, prepared as described in Example 8.

DMA: N,N-dimethylacetamide

Solutol: 15-hydroxystearic acid polyethylene glycol ester

Saline: normal saline

PEG: polyethylene glycol 400

Animal: C57BL/6 female mice

Animal administration and sample collection:

The Compound 1 used in the test was dissolved in 10% DMA + 10% (30% solutol) + 80% saline to prepare a solution, and the non-deuterated compound was dissolved in 10% DMA + 10% PEG + 80% saline to prepare a solution. C57BL/6 female mice were administered intravenously with Compound 1 at a dose of 5.0 mg/kg. The time points for blood sampling were: 5 min, 15 min, 30 min, 1 h, 2 h, 4 h, 6 h, 8 h, and 24 h. About 100 μL of blood was collected from the venous plexus, and the blood sample was placed in an anti-coagulation tube containing EDTA-K2. The blood sample was centrifuged at 8000 rpm for 6 minutes at 4 °C to obtain a plasma sample, wherein the plasma must be prepared within 30 minutes after blood sampling, and stored in -80 °C refrigerator before plasma testing.

Sample analysis method:

The sample (-80 °C) to be tested was taken out from the refrigerator, melted at room temperature and vortexed for 5 minutes; 10 μL of plasma samples of 3 different individuals were precisely pipetted into 1.5 mL centrifuge tubes (for standard curve of plasma, 30 μL); 200 μL of 100 ng/mL internal standard working solution was added, and mixed well; vortexed for 5 min, then centrifuged for 5 min (12000 rpm); precisely pipetted 50 μL of supernatant into a 96-well plate to which was added 150 μL/well of water in advance; vortexed and mixed for 5 min. The injection volume was 15 μL, and LC-MS/MS measurement was carried out.

Data processing method:

The concentrations of test substance were used from the analysis results of AB Analyst 1.6.3. Microsoft Excel was used to calculate the parameters such as mean, standard deviation, and coefficient of variation (those directly output from Analyst 1.6.3 were not calculated). The PK parameters were calculated using Pharsight Phoenix 6.1 software NCA.

Results:

Table 4: In vivo PK parameters in C57BL/6 mice (I.V, Mean, female, 3 groups, n = 3)

| Compound No. | Dosage | $t_{z\ 1/2}$ (h) | $AUC_{last}$ (h*ng/mL) | $AUC_{INF_obs}$ (h*ng/mL) | $V_{z_obs}$ (L/kg) | $Cl_{_obs}$ (L/h/kg) |
|---|---|---|---|---|---|---|
| 1 | 5 mg/kg | 3.70 | 2038 | 2288 | 11.7 | 2.19 |
| Compound R | 5 mg/kg | 1.81 | 1115 | 1138 | 12 | 4.39 |

From the above results, it could be seen that the Compound 1 of the present invention had a lower clearance rate and a higher exposure amount, thereby showing excellent pharmacokinetic properties, good drugability, and great clinical application value.

**Test example 4: Stability test of Crystalline Form A of the present invention**

Method: Crystalline Form A was left open at high temperature and in high humidity conditions, and XRPD patterns were compared to determine impurity contents. The data in Table 5 below were obtained.

Table 5: Study on Stability of Crystalline Form A

| Placement conditions | XRPD |
|---|---|
| 0-Day | Crystal Form A, Figure 1 |
| High temperature, 105°C (left open, 3 days) | Consistent with the 0-day XRPD pattern. Figure 6 |
| RH 92.5% (left open, 3 days) | Consistent with the 0-day XRPD pattern. Figure 7 |
| Placement conditions | Impurity, % |
| 0-Day | 0.74% |
| RH 92.5% (left open, 10 days) | 0.99% |

**Test example 5: Stability test of Crystalline Form B of the present invention**

Method: Crystalline Form B was left open for 10 days at high temperature and in high humidity conditions. The properties, measured content and impurities of the Crystalline Form B were observed, and the XRPD patterns were compared to obtain the data in Table 6.

Table 6: Stability investigation of Crystalline Form B

| Placement conditions | Properties | Impurity, % | Content, % | XRPD |
|---|---|---|---|---|
| 0-Day | Off-white powder | 0.17% | 99.6% | Crystal Form B, Figure 3 |
| High temperature, 60°C (left open, 10 days) | Off-white powder | 0.16% | 99.4% | Consistent with the 0-day XRPD pattern. Figure 8 |
| RH 92.5% (left open, 10 days) | Off-white powder | 0.15% | 99.3% | Consistent with the 0-day XRPD pattern. Figure 9 |

**Test example 6: Study of in vivo pharmacodynamics of the compound of the present invention on subcutaneous homograft model with colon cancer cell CT26 in mouse**

Test substance: Compound 1 of the present invention, which structure was shown in the foregoing.
Animals, cells, reagents and instruments:

CT26 cells, Balb/C mice.

DMA: N,N-dimethylacetamide

Solutol: 15-hydroxystearic acid polyethylene glycol ester

MC: methyl cellulose

Experiment Method:

(1) Construction and grouping of tumor-bearing mice

CT26 cells were cultured in RPMI1640 medium containing 10% fetal bovine serum. CT26 cells in exponential growth phase were collected, and resuspended in PBS to a suitable concentration for subcutaneous tumor inoculation in mice.
0.1 mL of cell suspension (cells suspended in PBS) containing about $5\times10^5$ CT26 cells was subcutaneously inoculated into the skin of the right back of female Balb/C mice. The mice were grouped and administrated when the average tumor volume reached about 100 $mm^3$. Grouping method: weighing animals before administration, and measuring tumor volume. According to tumor volume, the mice were grouped using block design, 9 mice in each group.

(2) Dosing regimen

Administration was performed according to Table 7 below.

Table 7: Administration mode

| Group | Dose (mg/kg) | Route of administration | Frequency of administration | Period of administration |
|---|---|---|---|---|
| Vehicle control (10% DMA + 20% (30% Solutol) + 70% (0.25% MC) | 0 | PO. | Bid | 10 days |
| Compound 1 | 150 | PO. | Bid | 10 days |
| PO.: oral administration; Bid .: administrated twice per day; | | | | |

(3) Test index for study

The animals' health and death were monitored daily, their body weights and tumor volumes were measured twice a week, and samples were collected after the last dose. The curative effect of tumor volume was evaluated by TGI%, wherein relative tumor suppression rate TGI (%) was: TGI = 1-T/C (%). T/C% was the relative tumor increment rate, that is, the percentage of tumor volume in the treatment group relative to that of the control group at a specific time point. T and C were the relative tumor volume (RTV) of the treatment group and the control group at a specific time point, respectively. The calculation formula was: T/C% = $T_{RTV}/C_{RTV} \times 100\%$ ($T_{RTV}$: the average RTV of the treatment group; $C_{RTV}$: the average RTV of the vehicle control group; RTV = $V_t/V_0$, $V_0$ was the tumor volume of the animal at the beginning of grouping, and Vt is the tumor volume of the animal after treatment.

(4) Results

Table 8: Effects of Compound 1 on tumor growth in CT26 tumor-bearing mice

| Group | Number of animals | Tumor volume ($mm^3$) | TGI (%)[a] |
|---|---|---|---|
| Vehicle control (10% DMA + 20% (30% Solutol) + 70% (0.25% MC) | 9 | 687.98 ± 126.96 | -- |
| Compound 1 | 9 | 414.32 ± 52.44 | 38 |
| Note: [a:] TGI: tumor volume inhibition rate. | | | |

From the test results in Table 8, it could be seen that the compound of the present invention had a certain inhibitory effect on the CT26 cell transplanted tumor model, indicating that the compound of the present invention had certain clinical prospects in immunotherapy of tumors.

**Claims**

**1.** A compound of Formula I, a pharmaceutically acceptable salt thereof and a stereoisomer thereof:

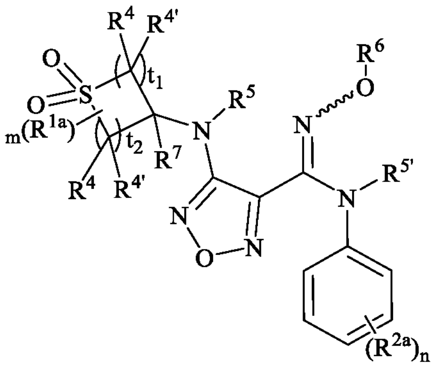

I

wherein,  represents a cis isomer, a trans isomer, or a mixture of cis and trans isomers;
$R^4$ and $R^{4'}$ are independently selected from hydrogen, deuterium, $C_{1-6}$ alkyl, or $C_{1-6}$ alkyl deuterated by one or more deuterium atoms;
$R^5$, $R^{5'}$, $R^6$, $R^7$ are independently selected from hydrogen or deuterium;
each $R^{1a}$ is independently selected from hydrogen, deuterium, halogen, $C_{1-6}$ alkyl, or $C_{1-6}$ alkyl deuterated by one or more deuterium atoms;
each $R^{2a}$ is independently selected from hydrogen, deuterium, halogen, $C_{1-6}$ alkyl, or $C_{1-6}$ alkyl deuterated by one or more deuterium atoms;

m is 0, 1 or 2;
n is 0, 1, 2, 3, 4 or 5;
$t_1$ and $t_2$ are independently 0, 1, 2, 3, and $t_1$ and $t_2$ are not 0 at the same time;

with the proviso that the compound of Formula I contains at least one deuterium atom.

**2.** The compound of Formula I according to Claim 1, a pharmaceutically acceptable salt thereof and a stereoisomer thereof, further having a structure represented by Formula II:

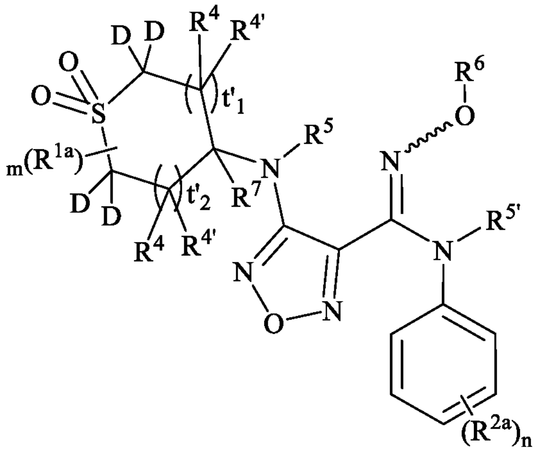

II

wherein, $\sim$ represents a cis isomer, a trans isomer, or a mixture of cis and trans isomers;
$R^4$ and $R^{4'}$ are independently selected from hydrogen or deuterium;
$R^5$, $R^{5'}$, $R^6$, $R^7$ are independently selected from hydrogen or deuterium;
each $R^{1a}$ is independently selected from hydrogen or $C_{1-6}$ alkyl;
each $R^{2a}$ is independently selected from hydrogen, deuterium, halogen or $C_{1-6}$ alkyl;
m is 0, 1 or 2;
n is 0, 1, 2, 3, 4 or 5;
$t'_1$ and $t'_2$ are each independently 0, 1 or 2.

**3.** The compound according to Claim 2, a pharmaceutically acceptable salt thereof and a stereoisomer thereof:

wherein, $\sim$ represents a cis isomer, a trans isomer, or a mixture of cis and trans isomers;
$R^4$ and $R^{4'}$ are independently selected from hydrogen or deuterium;
$R^5$, $R^{5'}$, $R^6$, $R^7$ are independently selected from hydrogen;
each $R^{1a}$ is independently selected from hydrogen;
each $R^{2a}$ is independently selected from hydrogen, deuterium or halogen;
m is 0;
n is 0, 1 or 2.
$t'_1$ and $t'_2$ are each independently 0, 1 or 2.

**4.** The compound according to Claim 3, a pharmaceutically acceptable salt thereof and a stereoisomer thereof, the compound being selected from the group consisting of:

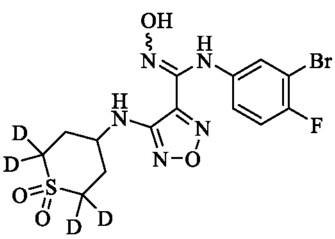
,
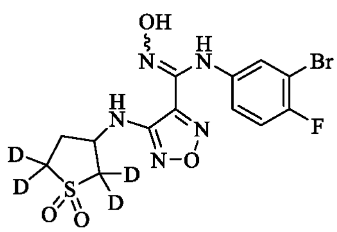
,

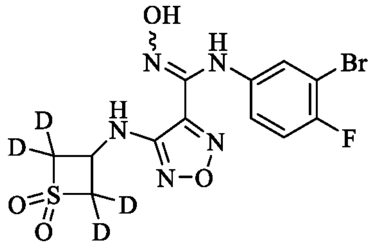
,
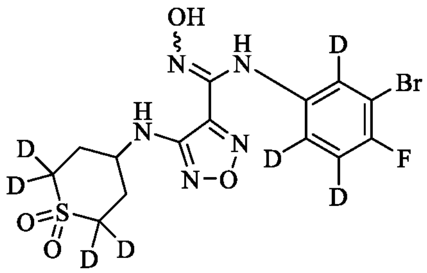
.

**5.** The compound according to Claim 1, a pharmaceutically acceptable salt thereof and a stereoisomer thereof, the compound being selected from the group consisting of:

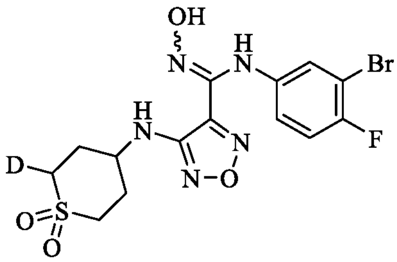
,
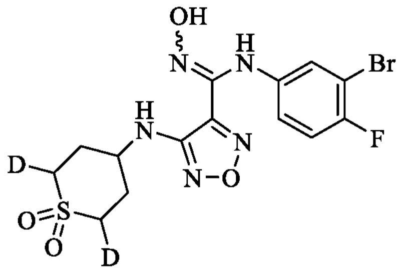
,

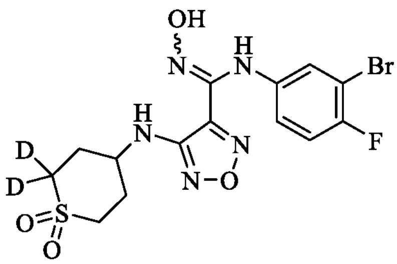
,
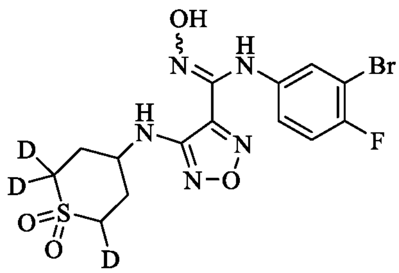
.

**6.** The compound according to any one of Claims 1 to 5, a pharmaceutically acceptable salt thereof and a stereoisomer thereof, which has at least 65% deuterium enrichment.

**7.** The compound according to any one of Claims 1 to 5, a pharmaceutically acceptable salt thereof and a stereoisomer thereof, which has at least 75% deuterium enrichment.

**8.** The compound according to any one of Claims 1 to 5, a pharmaceutically acceptable salt thereof and a stereoisomer thereof, which has at least 90% deuterium enrichment.

**9.** The compound according to any one of Claims 1 to 5, a pharmaceutically acceptable salt thereof and a stereoisomer thereof, which has at least 95% deuterium enrichment.

**10.** The compound according to any one of Claims 1 to 5, a pharmaceutically acceptable salt thereof and a stereoisomer thereof, which has at least 98% deuterium enrichment.

**11.** Crystalline Form A of Compound 1 as shown in the following formula,

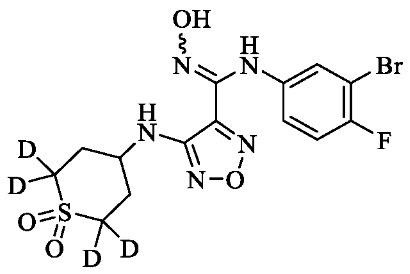

Compound 1,

when using CuKa radiation, which shows an X-ray powder diffraction pattern including peaks at the following diffraction angles (2θ): 8.8±0.2°, 18.0±0.2°, 19.5±0.2°, 22.4±0.2°, 23.1±0.2°, 23.8±0.2°, 27.8±0.2°.

**12.** Crystalline Form A of Compound 1 as shown in the following formula,

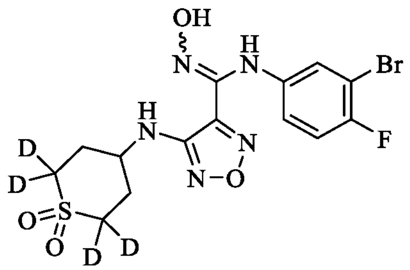

Compound 1,

when using CuKa radiation, which shows an X-ray powder diffraction pattern including peaks at the following diffraction angles (2θ): 8.8±0.2°, 11.9±0.2°, 17.6±0.2°, 18.0±0.2°, 18.7±0.2°, 19.5±0.2°, 22.4±0.2°, 23.1±0.2°, 23.8±0.2°, 25.1±0.2°, 26.8±0.2°, 27.8±0.2°, 30.0±0.2°.

**13.** Crystalline Form A of Compound 1 as shown in the following formula,

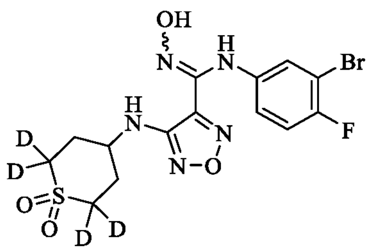

Compound 1,

when using CuKa radiation, which shows substantially the X-ray powder diffraction pattern as shown in Figure 1.

**14.** Crystalline Form B of Compound 1 as shown in the following formula,

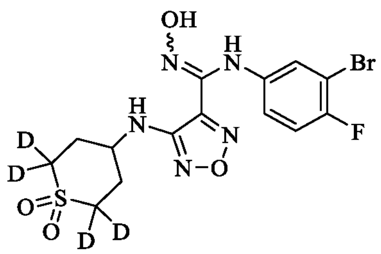

Compound 1,

when using CuKa radiation, which shows an X-ray powder diffraction pattern including peaks at the following diffraction angles (2θ): 16.2±0.2°, 18.0±0.2°, 21.1±0.2°, 21.6±0.2°, 21.8±0.2°, 23.7±0.2°, 23.9±0.2°, 26.2±0.2°.

**15.** Crystalline Form B of Compound 1 as shown in the following formula,

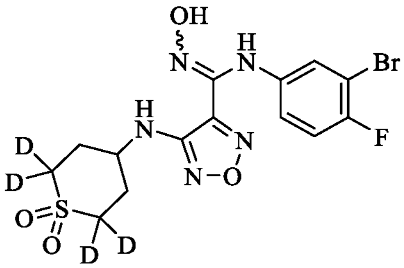

Compound 1,

when using CuKa radiation, which shows an X-ray powder diffraction pattern including peaks at the following diffraction angles (2θ): 10.7±0.2°, 15.8±0.2°, 16.2±0.2°, 16.7±0.2°, 18.0±0.2°, 19.3±0.2°, 20.1±0.2°, 21.1±0.2°, 21.6±0.2°, 21.8±0.2°, 22.5±0.2°, 23.2±0.2°, 23.7±0.2°, 23.9±0.2°, 26.2±0.2°.

**16.** Crystalline Form B of Compound 1 as shown in the following formula,

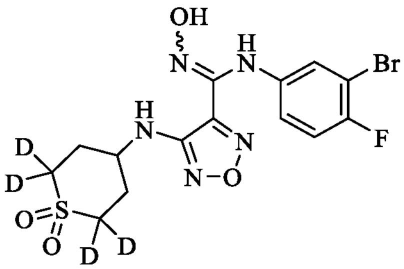

Compound 1,

when using CuKa radiation, which shows substantially the X-ray powder diffraction pattern as shown in Figure 3.

**17.** A method for preparing a compound as shown in Formula M-05, comprising the following steps:

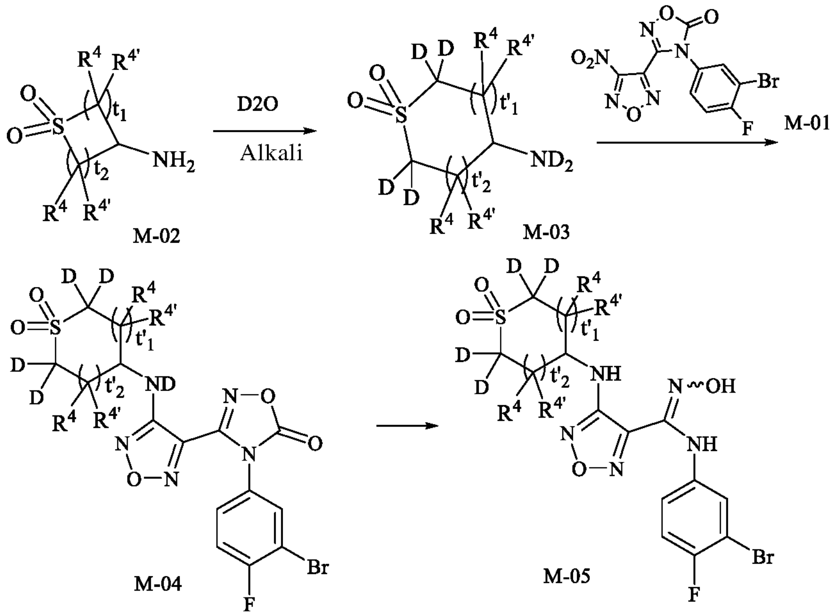


wherein, $R^4$, $R^{4'}$, $R^5$, $R^{5'}$, $t_1$, $t_2$, $t_1'$, $t_2'$ are as defined in Claim 1;
Step 1: M-02 is dissolved in deuteroxide, an alkali is added, the reaction is carried out at reflux for more than 2 hours, the deuteration rate is detected by using NMR, and deuteroxide is added repeatedly until the deuteration rate detected by NMR is above 75% to obtain a mother liquor of M-03;
Step 2: under the protection of nitrogen, a solution of M-01 in a solvent selected from the group consisting of 2-methyltetrahydrofuran, tetrahydrofuran, dimethylsulfoxide, ethyl acetate, acetonitrile, dimethylacetamide is added dropwise into a deuteroxide solution of M-03 and reacted for more than 1 hour, when the pH is measured to 7-12, anhydrous sodium carbonate, anhydrous sodium bicarbonate, anhydrous sodium hydroxide, imidazole, or N,N-diisopropylethylamine (DIEA) is added, reacted at 20-60°C for more than 10 hours, the reaction solution is subjected to suction filtration, then washed with water, acid aqueous solution (which is adjusted with hydrochloric acid, sulfuric acid, etc., to have an acidic pH, preferably pH 3-4) and anhydrous methanol in sequence, and dried to obtain M-04;
Step 3: under the protection of nitrogen, a solution of M-04 in tetrahydrofuran, dimethyltetrahydrofuran, dimethylformamide, or N,N-diisopropylethylamine is added, an aqueous solution of methylamine is added, and the reaction is carried out at reflux for 0.1 to 1 hour, the reaction solution is subjected to suction filtration, rinsed with ethyl acetate, the filtrate is washed with a mixed solution of concentrated hydrochloric acid and brine, , the organic phase is subjected to suction filtration and concentrated under reduced pressure to obtain M-05.

**18.** A pharmaceutical composition comprising the compound according to any one of Claims 1 to 10, a pharmaceutically acceptable salt thereof and a stereoisomer thereof, or the Crystalline Form according to any one of Claims 10 to 16, **characterized in** comprising one or more pharmaceutical carriers.

**19.** A use of the compound according to any one of Claims 1 to 10, a pharmaceutically acceptable salt thereof and a stereoisomer thereof, or the Crystalline Form according to any one of Claims 10 to 16 in manufacture of a medicament for treating a disease mediated by IDO abnormality.

**20.** The use according to Claim 19, wherein the disease mediated by IDO abnormality refers to an infectious disease, a nervous system disease, a cancer or a non-cancerous proliferative disease, and the infectious disease comprises diseases induced by influenza virus, hepatitis C virus (HCV), human papilloma virus (HPV), cytomegalovirus (CMV), E-B virus (EBV), polio virus, varicella zoster virus, Coxsackie virus, human immunodeficiency virus (HIV); the neurological disease comprises: Alzheimer's disease, depression; the cancer comprises lung cancer, squamous cell carcinoma, bladder cancer, gastric cancer, ovarian cancer, peritoneal cancer, breast cancer, breast ductal cancer, head and neck cancer, endometrial cancer, uterine cancer, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharynx cancer, multiple myeloma, leukemia, non-Hodgkin's lymphoma, colonic villous adenoma, melanoma, cell tumor and sarcoma, myelodysplastic syndrome.

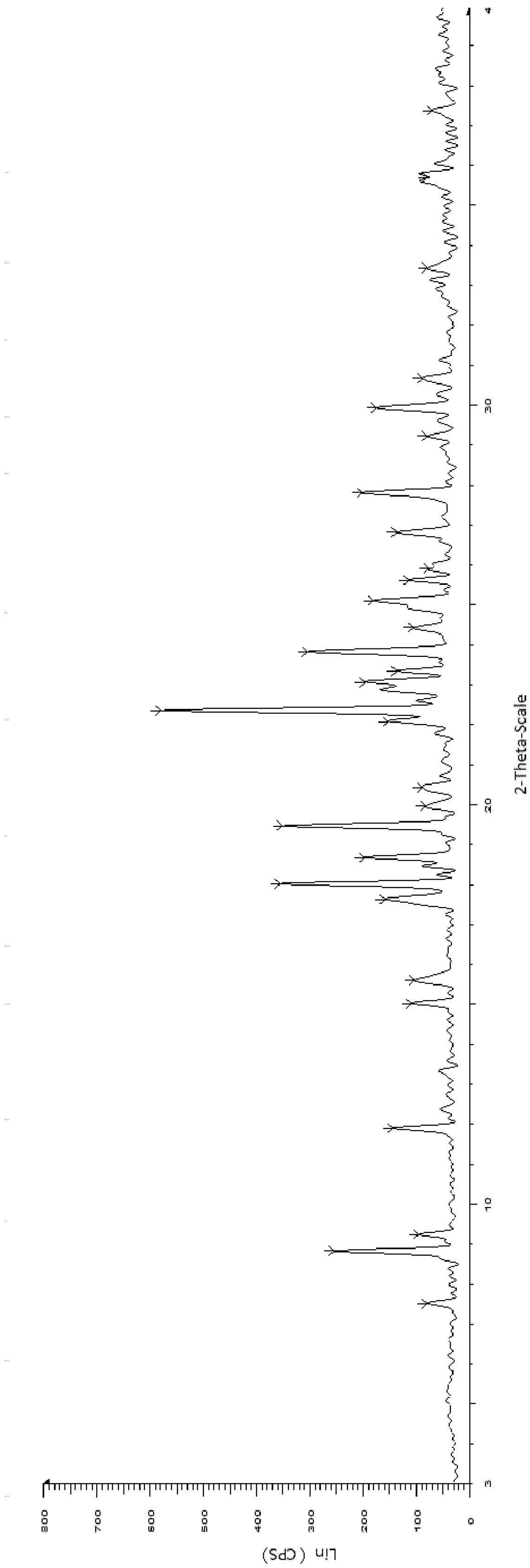
Lin (CPS)
0
100
200
300
400
500
600
700
800
2-Theta-Scale
3
10
20
30
4

Figure 1

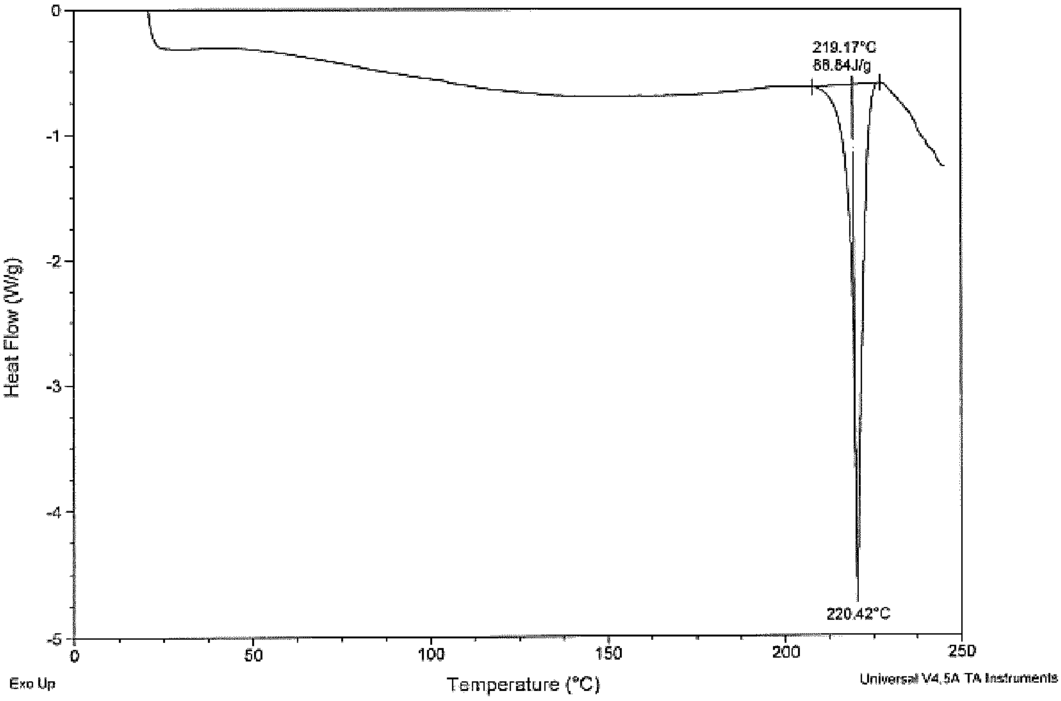
0
-1
-2
-3
-4
-5
Heat Flow (W/g)
219.17°C
88.84J/g
220.42°C
0
50
100
150
200
250
Exo Up
Temperature (°C)
Universal V4.5A TA Instruments

Figure 2

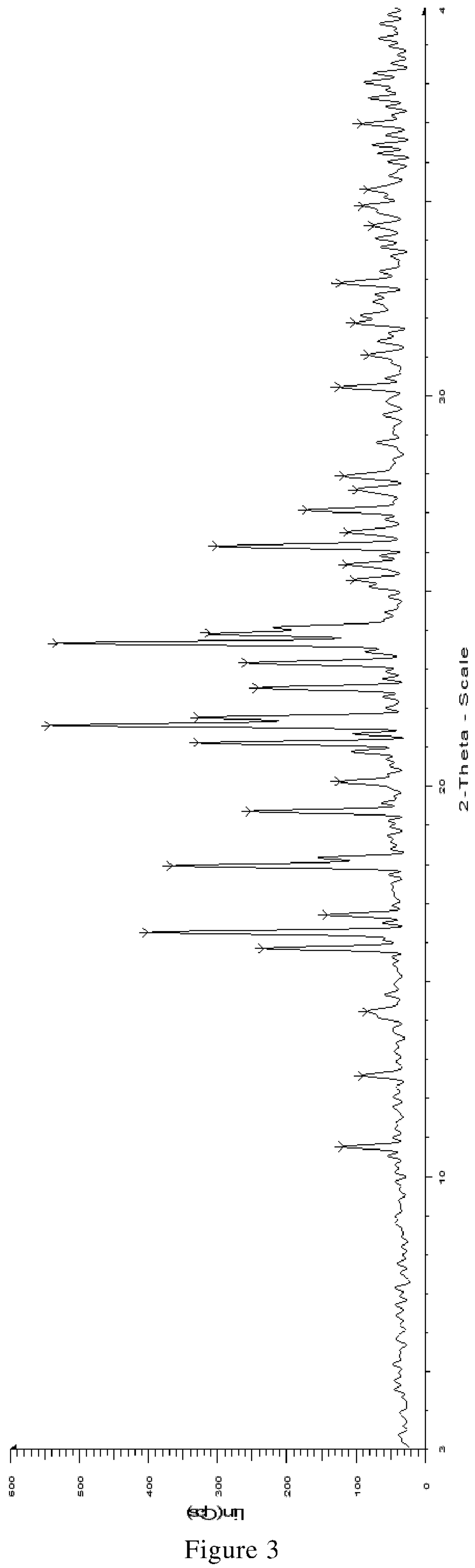


Figure 3

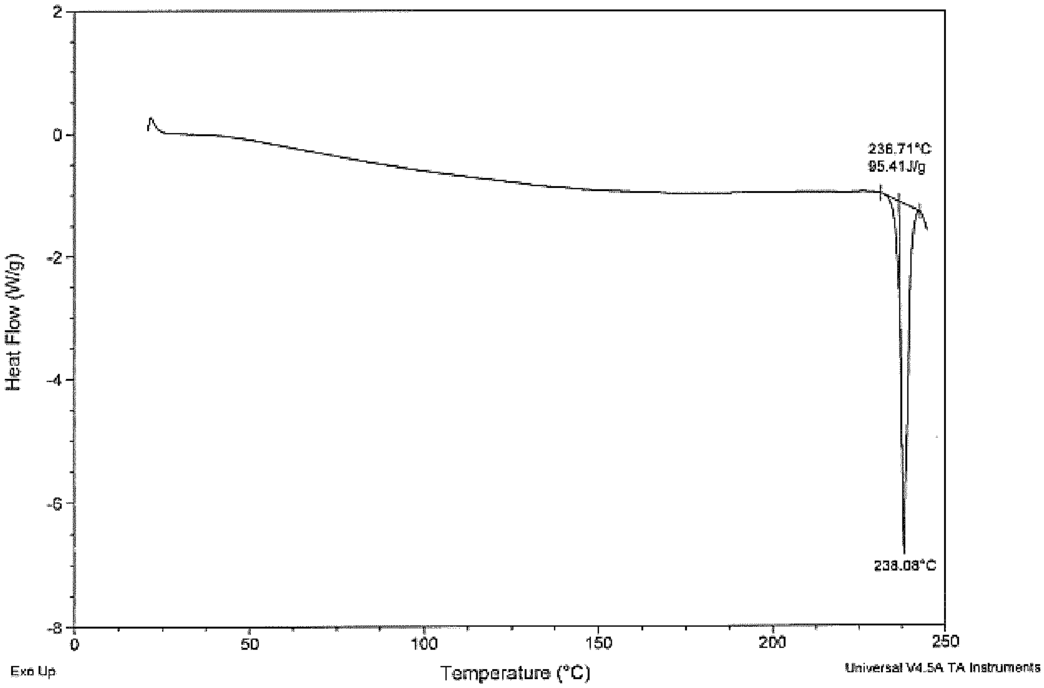


Figure 4

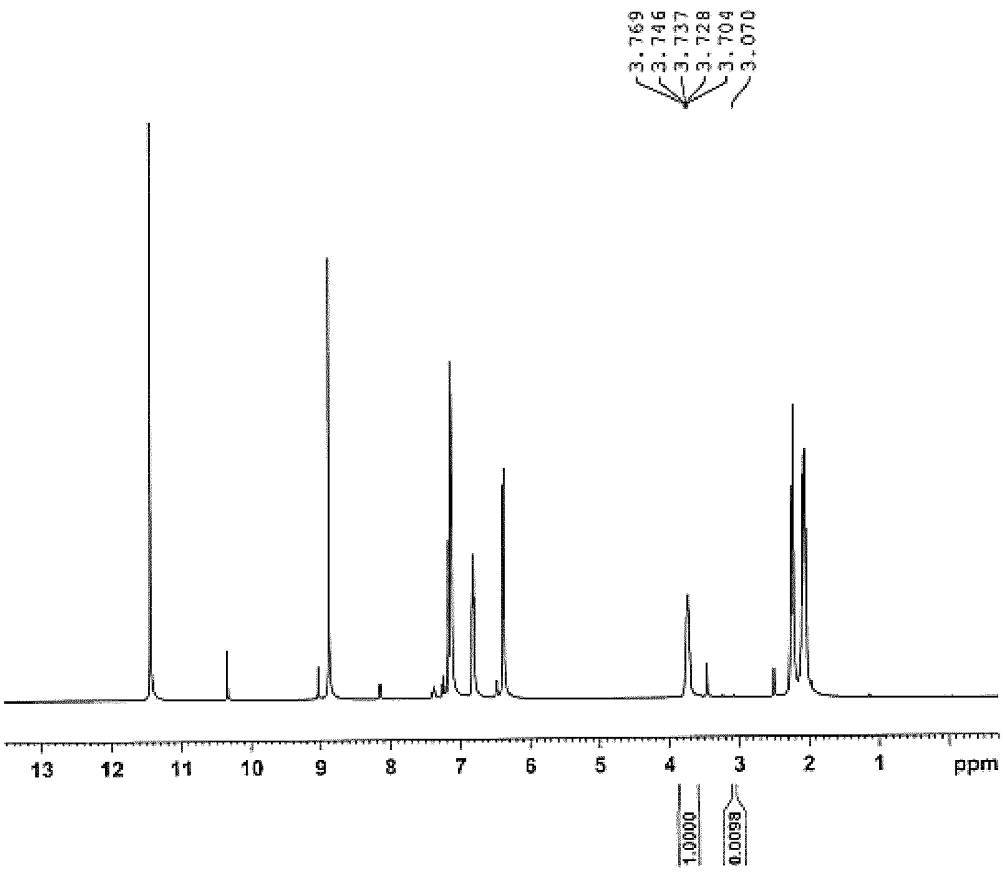


Figure 5

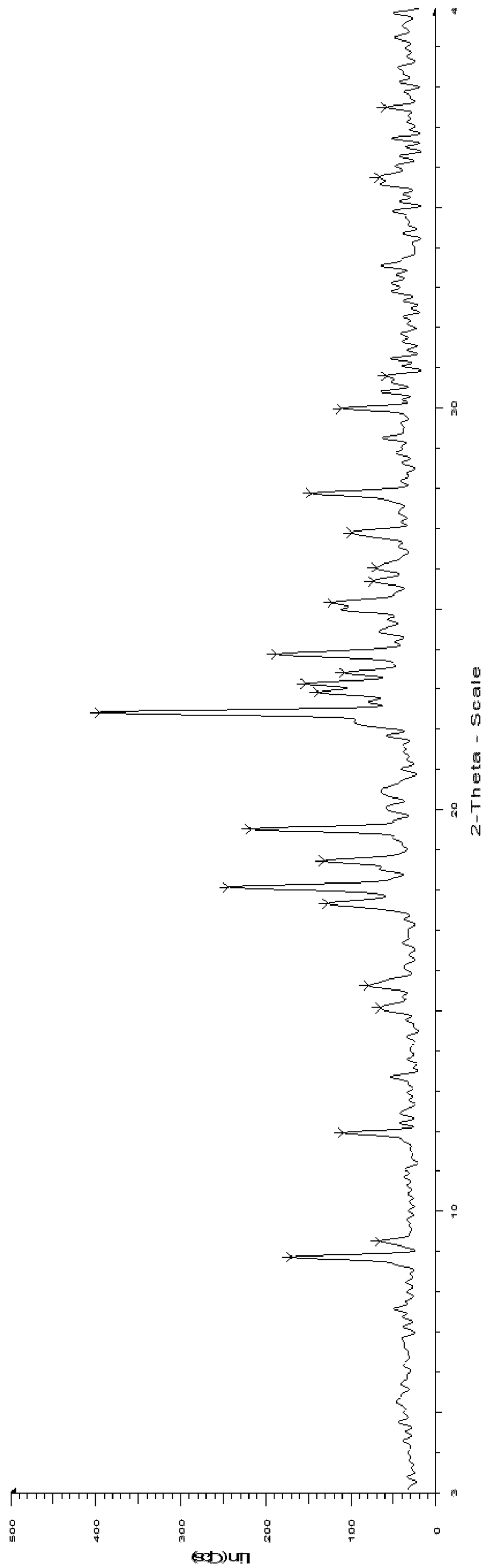


Figure 6

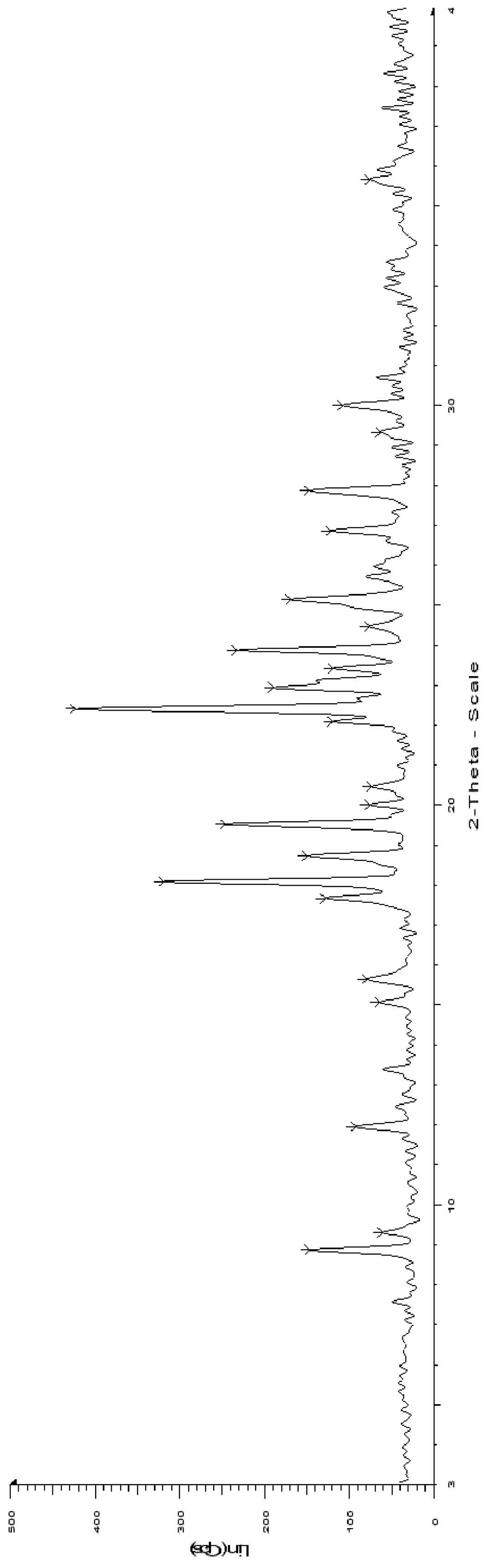


Figure 7

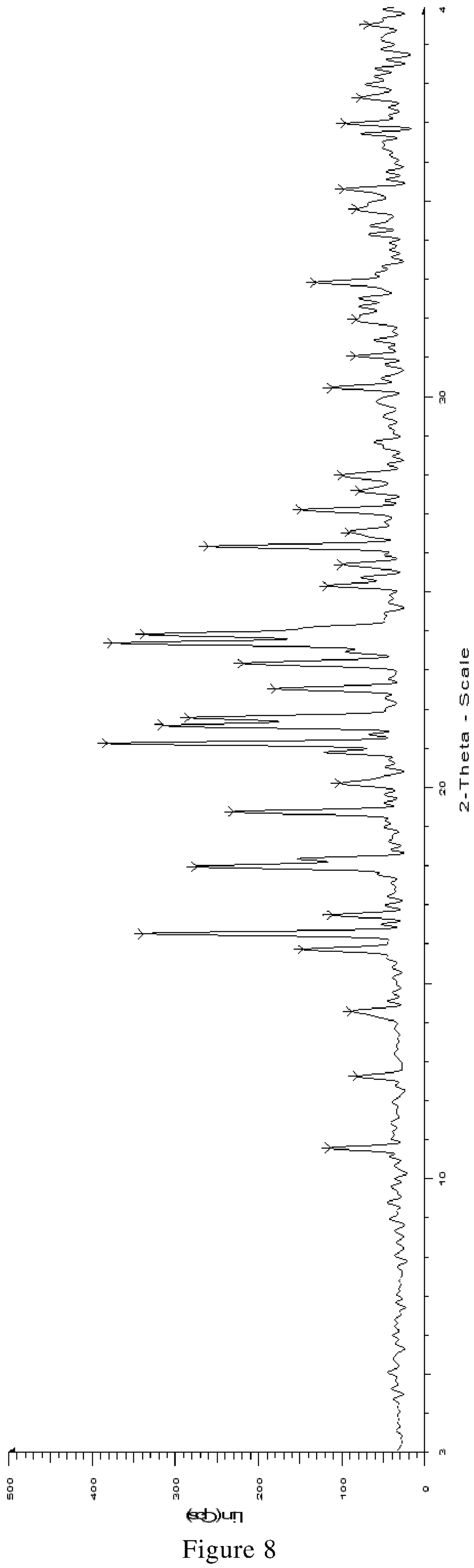


Figure 8

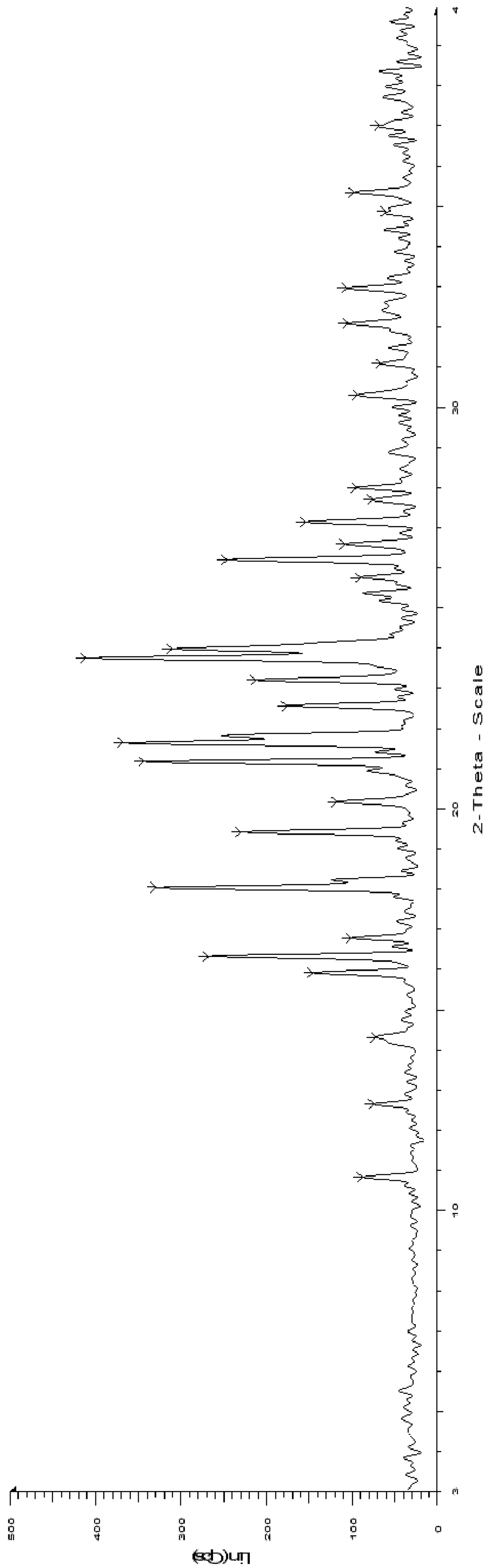


Figure 9

**INTERNATIONAL SEARCH REPORT**

International application No. **PCT/CN2018/103846**

**A. CLASSIFICATION OF SUBJECT MATTER**

C07D 413/12(2006.01)i

According to International Patent Classification (IPC) or to both national classification and IPC

**B. FIELDS SEARCHED**

Minimum documentation searched (classification system followed by classification symbols)

C07D413

Documentation searched other than minimum documentation to the extent that such documents are included in the fields searched

Electronic data base consulted during the international search (name of data base and, where practicable, search terms used)

CNABS; VEN; CNKI; 万方, WANFANG; ISI; STN: 南京药捷安康, 吴永谦, IDO, 吲哚胺, 双加氧酶, 氘代, deuterium, +dioxygenase, epacadostat, STN structure search

**C. DOCUMENTS CONSIDERED TO BE RELEVANT**

| Category* | Citation of document, with indication, where appropriate, of the relevant passages | Relevant to claim No. |
|---|---|---|
| Y | WO 2017143874 A1 (SHENZHEN TAJIRUI BIO PHARMACEUTICAL CO., LTD.) 31 August 2017 (2017-08-31) abstract, and description, pages 2-5 and 8 | 1-10 and 17-20 |
| Y | CN 106883194 A (JIANGSU HENGRUI MEDICINE CO., LTD. ET AL.) 23 June 2017 (2017-06-23) description, pages 3-5 and 9-10 | 1-10, 17-20 |
| A | CN 106256830 A (HINOVA PHARMACEUTICALS INC.) 28 December 2016 (2016-12-28) entire document | 1-20 |

☐ Further documents are listed in the continuation of Box C. ☑ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **30 September 2018** | **16 November 2018** |

| Name and mailing address of the ISA/CN | Authorized officer |
|---|---|
| **State Intellectual Property Office of the P. R. China**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2018/103846**

| Patent document cited in search report | Publication date (day/month/year) | Patent family member(s) | Publication date (day/month/year) |
|---|---|---|---|
| WO 2017143874 A1 | 31 August 2017 | CN 108602786 A | 28 September 2018 |
| CN 106883194 A | 23 June 2017 | None | |
| CN 106256830 A | 28 December 2016 | None | |

Form PCT/ISA/210 (patent family annex) (January 2015)